# EUROPEAN PATENT SPECIFICATION

(11) **EP 0 577 659 B1**
(45) Date of publication and mention of the grant of the patent: **17.01.1996**
(21) Application number: 92907057.1
(22) Date of filing: 28.03.1992
(51) Int. Cl.: C07C 69/96, A61K 49/00

(54) **CROSS-LINKING AGENT**
VERNETZUNGSAGENTIEN
AGENT DE RETICULATION

(30) Priority: 28.03.1991 GB 9106686; 08.07.1991 GB 9114678; 09.01.1992 GB 9200389
(43) Date of publication of application: 12.01.1994
(73) Proprietor: NYCOMED IMAGING AS, N-0401 Oslo 4 (NO)
(72) Inventor: KLAVENESS, Jo, N-0276 Oslo (NO); RONGVED, P l, N-1454 Hellvik (NO); STRANDE, Per, N-0755 Oslo (NO)
(74) Representative: Marsden, John Christopher
(86) International application number: EP9200717
(87) International publication number: WO9217436

(56) References cited:
- EP-A- 0 083 185

## Description

This invention relates to novel crosslinking agents, more particularly to crosslinking agents capable of generating biodegradable crosslinking groups.

The use of crosslinking agents in fields such as protein and polymer chemistry is widespread and well known, e.g. for investigative or stability-enhancing purposes. The possibility of deliberately introducing biodegradable crosslinking groups has not hitherto been disclosed,, but has been found by us to possess a substantial number of utilities, for example in the preparation of biodegradable polymers (e.g. as described in our copending International Patent Application No. PCT/EP91/01751), in the attachment of drugs or agricultural chemicals to polymer systems (e.g. to provide delayed release delivery systems), and in the preparation of stabilised but biodegradable and therefore rapidly eliminable ultrasound contrast agents based on microbubbles encapsulated by crosslinked liposomes or crosslinked proteins (e.g. as described in our copending British Patent Applications Nos. 9106673.8 and 9106686.0 respectively) or on microparticles of crosslinked carbohydrates, X-ray contrast agents, polypeptides and proteins (e.g. as described in our copending British Patent Application No. 9114570.6); the contents of the specifications of the aforementioned applications are herein incorporated by reference.

The crosslinking agents of the invention are characterised in that they contain, or are capable of generating during crosslinking, methylene diester or diamide groups in which the ester or amide residues are derived from a range of carbon, sulphur and phosphorus acids. Such groups are particularly rapidly degraded by common esterase enzymes but are stable in the absence of enzymes.

A small number of compounds falling within this definition have previously been described in the literature and these specific compounds per se are excluded from the scope of the invention. Thus, for example, US-A-2341334 describes methylene dimethacrylate, ethylidene dimethacrylate and butylidene dimethacrylate as being copolymerisable with ethylenically unsaturated monomers such as vinyl acetate, methyl methacrylate or styrene; DD-A-95108 describes the preparation of benzylidene dimethacrylate and 2,2,2-trichloroethylidene dimethacrylate; US-A-2839572 describes the preparation of a number of alkenylidene crotonates such as allylidene dicrotonate, methallylidene dicrotonate and 2-chloroallylidene dicrotonate; US-A-2568501 describes the preparation of heptafluorobutylidene diacrylate; propylidyne trimethacrylate is described by Kimura H. in J. Osaka Univ. Dent. Sch., 20 (1980), pp. 43-49; propylidyne triacrylate is described by Cox R.J. in Polym. Prep. (Am. Chem. Soc., Div. Polym. Chem.) 29 (1988), pp. 122-123; and allylidene diacrylate and allylidene dimethacrylate are described by Arbuzova A. et al. in Zh. Obshch. Khim. 26 (1956), pp. 1275-1277. Other disclosures of the use of certain of these compounds as monomers, comonomers or crosslinking agents include Szymczak T.J. et al. in Modern Plastics (August 1974), pp. 66-68 and in West. Elec. Eng. 18 (1974), pp. 26-30; DE-A-1104700; and FR-A-2119697. Crosslinking involving the use of N,N'-methylenebis(acrylamide), and in certain cases N,N'-methylenebis(methacrylamide), is described in, for example, US-A-4 743 267, US-A-4 962 170, US-A-5011864, EP-A-0383124, EP-A-0383126, CA-A-1249952; and by Capek et al., Makromol. Chem. 191 (1990), pp. 121-138 and 192 (1991), pp. 2031-2040, and Latha et al., J. Appl. Polym. Sci. 43 (1991), pp. 1159-1163.

There is no suggestion in any of the above prior art that the methylene di(carboxylic ester) or N,N'-di(carboxamide) groupings resulting from polymerisation or crosslinking might be biodegradable; indeed, the introduction of crosslinking groups of this type is generally seen as conveying enhanced rigidity and/or stability. The present invention accordingly embraces the use of these known compounds in the preparation of biodegradable crosslinked structures.

It should be noted that in the prior art crosslinking methylene di(carboxylic ester) groups are invariably present as simple carbon-attached ester groups, as a consequence of their introduction by free radical propagated reactions of e.g. alkylidene diacrylates or dimethacrylates.

Subject to the foregoing disclaimer, the novel compounds of the present invention may be regarded as crosslinking agents containing a group of formula 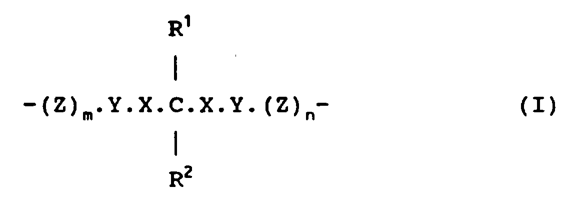 [in which each X, which may be the same or different, is selected from -O-, -S- and -NR-, where R represents a hydrogen atom or an organic group; each Y, which may be the same or different, represents carbonyl, thiocarbonyl, sulphonyl or phosphoryl (i.e. a group of formula 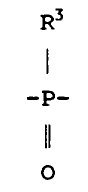 where R³ is a hydrogen atom or an organic group) or a similar acid-forming group; each Z, which may be the same or different, is selected from -O-, -S- and -NR-, where R represents a hydrogen atom or an organic group; m and n, which may be the same or different, are each zero or 1; and R¹ and R, which may be the same or different, are each selected from hydrogen atoms, monovalent organic groups and groups of formula -X.Y.(Z)ₘ- as hereinbefore defined, or R¹ and R together form a divalent organic group] or containing a group adapted to generate a group of formula (I) upon reaction with a reagent or substrate containing a species H.X.Y.(Z)ₘ- or a reactive derivative thereof.

The term "crosslinking" as used herein denotes the introduction of any desired proportion of crosslinking groups and thus generally embraces the preparation of copolymers containing linkages of formula (I).

Organic groups represented by R, R¹, R and R³ may, for example, each be a hydrocarbyl or heterocyclic group, for example having 1-20 carbon atoms, e.g. an aliphatic group such as an alkyl or alkenyl group (preferably having up to 10 carbon atoms), a cycloalkyl group (preferably having up to 10 carbon atoms), an araliphatic group such as an aralkyl group (preferably having up to 20 carbon atoms), an aryl group (preferably having up to 20 carbon atoms) or a heterocyclic group having up to 20 carbon atoms and one or more heteroatoms selected from O,S and N; such a hydrocarbyl or heterocyclic grouping may carry one or more substituents such as halogen atoms or groups of the formulae -NR⁴R⁵, -CONR⁴R⁵,-OR⁶,-SR⁶ and -COOR⁷ (where R⁴ and R⁵, which may be the same or different, are hydrogen atoms, acyl groups or hydrocarbyl groups as defined for R, R¹, R and R³; R⁶ is a hydrogen atom or an acyl group or a group as defined for R, R¹, R and R³; and R⁷ is a hydrogen atom or a group as defined for R, R¹, R and R³). Where R¹ and R represent a divalent grouping, this may be an alkylene, alkenylene, alkylidene or alkenylidene group (preferably having up to 10 carbon atoms) which may carry one or more substituents as defined above. In general R,R¹,R and R³ are preferably H or small groups such as C₁₋₄ alkyl groups.

Aliphatic groups R, R¹, R and R³ may be straight or branched, saturated or unsaturated, and include, for example, alkyl and alkenyl groups such as methyl, ethyl, isopropyl, butyl and allyl. Araliphatic groups include (monocarbocyclic aryl) alkyl groups such as benzyl. Aryl groups include mono- and bi-cyclic groups such as phenyl, tolyl and naphthyl. Heterocyclic groups include 5- and 6-membered rings preferably containing a single heteroatom, such as furyl, thienyl and pyridyl.

Possible substituents in hydrocarbyl groups R,R¹,R and R³ include hydroxyl, etherified hydroxyl (e.g. C₁₋₅ alkoxy such as methoxy), esterified hydroxyl (e.g. C₁₋₆ acyloxy such as acetoxy), etherified thiol, N-(C₁₋₆ alkyl)amino, N-(C₁₋₆ acyl)amino, N-(C₁₋₆ acyl)-N-(C₁₋₆ alkyl)amino, carbamoyl, N-(C₁₋₆ alkyl) carbamoyl and halogen. Aromatic rings may carry C₁₋₆ alkyl groups, e.g. as in tolyl groups. Substituents may be present in combination and thus, for example, N-acyl and N-alkyl groups may carry hydroxyl or etherified or esterified hydroxyl groups.

One preferred class of compounds according to the invention may be represented by the formula 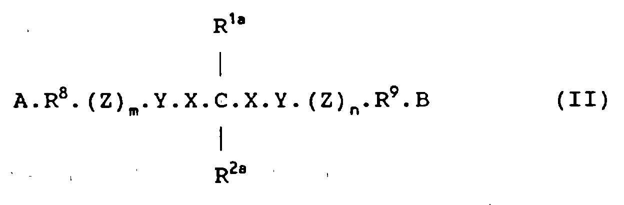 (wherein X,Y,Z, m and n are as hereinbefore defined; R^{1a} and R^{2a} are as defined for R¹ and R except that they may represent groups -X.Y.(Z)ₘ.R⁸.A or -X.Y.(Z)ₙ.R⁹.B rather than groups -X.Y.(Z)ₘ-; R⁸ and R⁹, which may be the same or different, represent divalent organic groups optionally interrupted by one or more heteroatoms and/or carrying one or more substituents containing heteroatoms; and A and B, which may be the same or different, optionally in conjunction with the groups R⁸ and R⁹ to which they are attached, represent functional groupings reactive with the species to be crosslinked; with the proviso that when both A.R⁸- and -R⁹.B represent optionally substituted lower alk-l-enyl groups, both of X represent -O- or -NR- and both of Y represent 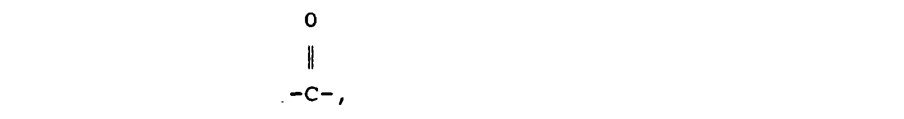 then at least one of m and n is 1).

A second preferred class of compounds according to the invention may be represented by the formula 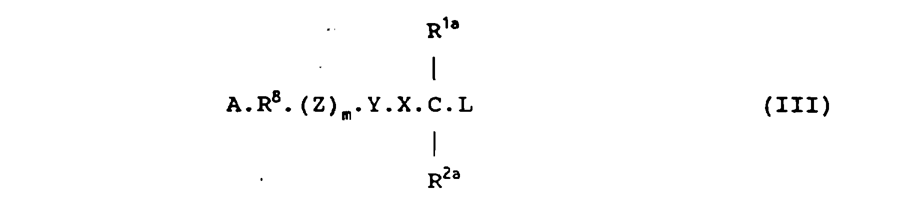 (wherein X,Y,Z, m, R^{1a}, R^{2a}, R⁸ and A have the above-defined meanings and L is a leaving group). Such compounds may be reacted with compounds of the formula

R¹⁰.(Z)ₙ.Y.X.H (IV)

(where X,Y,Z and n are as hereinbefore defined and R¹⁰ represents a hydrogen atom or an organic group), or appropriate reactive derivatives thereof (e.g. alkali metal salts of compounds of formula (IV) which are acids), to generate a biodegradable linkage of formula (I).

It will be appreciated that R¹⁰ may represent an organic group such that, for example, the compound (III) reacts to form a compound of formula (II) or a precursor therefor. Alternatively the group R¹⁰ may represent a substrate which is to be crosslinked; in addition to the -(Z)ₙ.Y.X.H substituent or reactive derivative thereof such a substrate will also possess a functional grouping reactive with -A or -R⁸A in formula (III).

The divalent organic groups R⁸ and R⁹ in the above formulae may, for example, be selected from alkylene and alkenylene groups (e.g. containing up to 30, more preferably up to 20, e.g. 1-10, carbon atoms), cycloalkylene groups (preferably having up to 10 carbon atoms), arylene groups (containing one or more aromatic rings and preferably having up to 20 carbon atoms), aralkylene groups (preferably having up to 20 carbon atoms and which may be bonded via the aryl and/or alkyl moieties - such aralkylene groups include, for example, two aryl groups joined by an alkylene chain), and heterocyclic groups (having one or more heteroatoms preferably selected from O, N and S and preferably having up to 20 carbon atoms). The groups may carry substituents, e.g. as set out above for R, R¹, R and R³ and/or substituents such as oxo or thio groups. The carbon chains may be interrupted by heteroatoms such as O, N, S or P, e.g. in conjunction with oxo substituents, to form linkages such as ether, ester, thioester or amide groups. The presence of disulphide linkages may also be advantageous by virtue of their inherent biodegradability.

It will be appreciated that groups R⁸ and/or R⁹ may be chosen so as to include one or more further groups of formula (I) and that the grouping - R⁸.A in formula (III) may be such that it terminates in a grouping 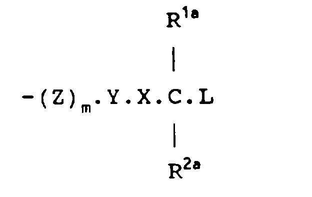 (where X, Y, Z, m, R^{1a}, R^{2a} and L are as hereinbefore defined) capable of generating a biodegradable linkage of formula (I).

The nature of functional groups A and B will clearly depend on the nature of the species which is to be crosslinked or otherwise reacted, in particular the nature of reactive functional groupings present therein. It will be appreciated that numerous complementary pairs of interacting functional groups are known in the art, e.g. as described by Beaumert et al. in "Crosslinking techniques" (Meth. Enzymol. 172 (1989), pp. 584-609) or in the Pierce Handbook and General Catalogue (1989), pp. 284-311.

Thus, for example, hydroxyl groups in substrates such as carbohydrates may be reacted as described in "Advances in Carbohydrate Chemistry and Biochemistry" ed. by R. Stuart Tipson and D. Horton, 33 (1976), pp. 11-109. Examples of appropriate functional groups for reacting with such substrates include halogen atoms such as chlorine or bromine, e.g. in the form of acyl halides such as alkanoyl or sulphonyl halides; sulphonyloxy groups, e.g. alkanesulphonyloxy groups such as mesyloxy groups and arenesulphonyloxy groups such as tosyloxy groups; α-halomethyl ester and keto groups; activated carboxyl groups such as symmetrical or mixed anhydrides; activated hydroxyl groups; activated alkenes, e.g. α,β-unsaturated esters, amides and ketones; conjugated diyne and enyne systems; epoxy groups; and acetal-forming aldehyde and ketone groups and derivatives thereof such as enol ethers or acetal or ketal groups.

Amino groups in substrates such as proteins may, for example, be reacted with functional groups such as activated carboxyl groups (e.g. N-hydroxysuccinimidyl derivatives, especially water solubility-enhanced sulphonated N-hydroxysuccinimidyl derivatives), imidoesters, nitroaryl halides, nitrene precursors (e.g. aryl azides such as phenylazido), carbene precursors (e.g. diazo compounds and diazirines), aldehydes, ketones, isocyanates, isothiocyanates, semicarbazides and thiosemicarbazides, epoxides, phenol esters (e.g. nitrophenol esters), acyl azides and hydrazines, haloformates, and acyl halides (e.g. alkanoyl chlorides or sulphonyl chlorides such as mesyl or tosyl chloride).

Carboxyl groups may, for example, be reacted with functional groups such as hydroxyl, mercapto, amino or diazo.

Sulfhydryl groups may, for example, be reacted with functional groups such as maleimides, sulphonated maleimides, α-halomethyl carbonyl derivatives (e.g. esters, amides or ketones), alkyl or aralkyl halides, nitrosoureas, s-triazines, aziridines and pyridyl disulphides.

Substrates containing ethylenically or acetylenically unsaturated carbon-carbon bonds (e.g. vinyl monomers such as vinyl acetate or styrene, or acrylic or methacrylic monomers such as acrylic acid, methacrylic acid, methyl acrylate, methyl methacrylate, acrylamide, methacrylamide, acrylonitrile, methacrylonitrile, hydroxyethyl methacrylate or hydroxypropyl methacrylate) may be copolymerised with compounds of formula (II) in which A and B comprise e.g. ethylenically unsaturated groups, for example under conditions appropriate for free radical polymerisation, to yield polymers containing biodegradable crosslinking groups of formula (I). It will be appreciated that in such circumstances the groups A and B may if desired form unsaturated groups in conjunction with R⁸ and R⁹ respectively; thus, for example, A.R⁸- and/or -R⁹.B may each represent optionally substituted vinyl groups.

Leaving groups L in compounds of formula (III) include halogen atoms such as chlorine or bromine and sulphonyloxy groups such as mesyloxy or tosyloxy.

Compounds in accordance with the present invention may be prepared by any convenient method. Thus, for example, one or two equivalents of a compound of formula

A.R⁸.(Z)ₘ.Y.X.H (V)

(where X, Y, Z, m, R⁸ and A are as hereinbefore defined, subject, if necessary and/or desired to A and any other reactive groups being protected), or a functional derivative thereof (e.g. a salt, for example an alkali metal salt such as the potassium or cesium salt of a compound (V) which is an acid), may be reacted with one equivalent of a compound of formula 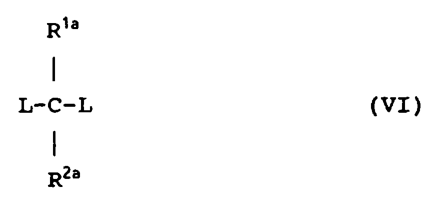 (where R^{1a}, R^{2a} and L are as hereinbefore defined) to yield compounds of formula (III) and symmetrical compounds of formula (II) respectively. Alternatively, if an unsymmetrical compound of formula (II) is required, one may, for example, react equivalent quantities, of a compound of formula (V), or a functional derivative thereof, and a compound of formula 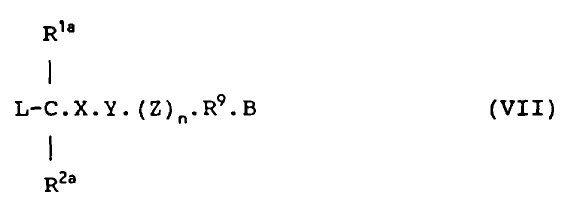 (where X, Y, Z, n, R^{1a}, R^{2a}, R⁹, B and L are as hereinbefore defined, subject if necessary and/or desired to B and any other reactive groups being protected). Such reactions will normally be carried out in solution, for example in a polar solvent such as dimethylformamide.

Symmetrical compounds of formula (II) in which R^{2a} represents a hydrogen atom, m and n are zero, each Y represents a carbonyl group and each X represents -O- may also be prepared by reacting a compound of formula

A.R⁸.CO.OH (VIII)

(where A and R⁸ are as hereinbefore defined, subject, if necessary and/or desired to A and any other reactive groups being protected) with an aldehyde of formula

R^{1a}.CHO (IX)

(where R^{1a} is as hereinbefore defined) in the presence of an acid catalyst such as hydrochloric acid; if desired water may be removed from the reaction mixture by azeotropic distillation.

Compounds of formula (III) in which L is a halogen atom may also be prepared by reaction of a compound of formula (V) as hereinbefore defined, particularly such a compound in which Y represents a carbonyl group and X represents -O-, with an aryl thioether of formula 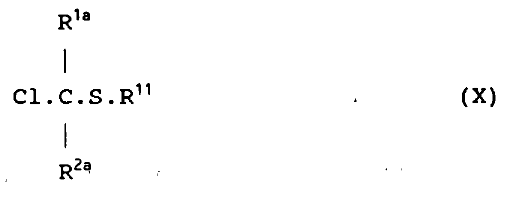 (where R^{1a} and R^{2a} are as hereinbefore defined and R¹¹ represents an aryl group such as phenyl), e.g. in a polar solvent such as dimethylformamide in the presence of a base such as pyridine, to yield a compound of formula 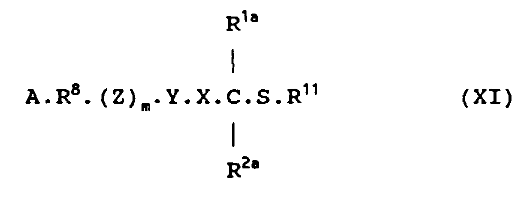 (wherein all the symbols are as hereinbefore defined) and halogenating this thioether, e.g. by reaction with sulfuryl chloride in a solvent such as dichloromethane or with bromine in a solvent such as carbon tetrachloride, to yield a compound (III) in which L is chlorine or bromine respectively.

Alternatively, compounds of formula (III) may be prepared by reaction of a compound of formula (V), as hereinbefore defined, with a chlorosulphate of formula 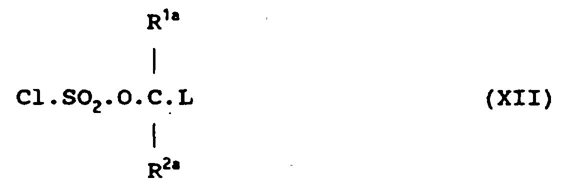 (wherein R^{1a}, R^{2a}, and L are as hereinbefore defined, L preferably being chlorine), e.g. using the method of Binderup et al. described in Synth. Comm. 14(9) (1984), pp. 857-864.

Protecting groups used in connection with A and B and any other reactive groups present may, for example, be those conventional in the art. Thus, for example, carboxyl groups may be protected using reductively cleavable ester groups such as benzyl, and hydroxyl groups may be protected using acid cleavable etherifying groups such as triphenylmethyl.

One may also prepare compounds of formulae (II) and (III) containing precursors for the desired A.R⁸- (and/or -R⁹.B groups where appropriate) and subsequently convert such precursor groups to the desired reactive groupings. Thus, for example, compounds in which A and/or B represent epoxide groups may be prepared by oxidation of precursors containing appropriately positioned (e.g. terminal) ethylenically unsaturated bonds (e.g. using an oxidising agent such as metachloroperbenzoic acid), or by reacting compounds containing appropriately positioned hydroxyl groups (e.g. phenolic hydroxyl groups) with reagents such as epichlorohydrin; compounds in which A.R⁸- and/or -R⁹.B represent enol ether groups may be prepared by, for example, acid-catalysed elimination from corresponding acetals or ketals. Hydroxyl group-containing precursors may also be activated by, for example, reaction with sulphonyl halides such as mesyl or tosyl chloride to generate reactive leaving groups such as mesylate or tosylate or with α,β-unsaturated alkenoyl halides such as acryloyl chloride to generate α,β-unsaturated esters.

Compounds of formula (VII) in which L represents a halogen atom may, for example, be prepared by reacting compounds of formulae 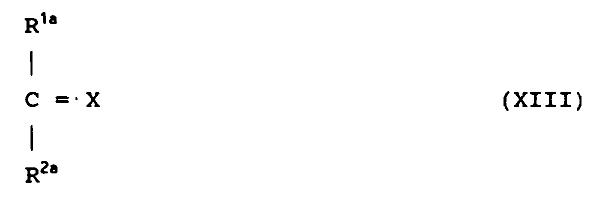 and

Hal.Y.(Z)ₙ.R⁹.B (XIV)

(where Hal represents a halogen atom and the remaining symbols have the above-defined meanings), e.g. in the presence of a base such as pyridine.

As hereinbefore indicated, the invention embraces the use of all compounds containing a group of formula (I) or capable of reacting to generate such a group, including compounds of formula (II) subject to the aforegoing proviso regarding the definitions of X, Y, m, n, R⁸, R⁹, A and B, in the prepartion of substrates containing biodegradable crosslinking groups. Such uses include, for example, the previously mentioned covalent stabilisation of a range of ultrasound contrast agents, thereby enhancing the duration of attenuative activity of such agents in vivo while permitting their ready subsequent elimination from the body, and the preparation of polymers useful in the manufacture of, for example, surgical implants, soft tissue prostheses, sponges, films, wound dressings, flexible sheets, containers, plasticisers, delayed release formulations for drugs (e.g. steroids, contraceptives, antibacterials, narcotic antagonists and anti-tumour drugs) and agricultural chemicals (e.g. weed killers), and polymer particles incorporating diagnostic agents (e.g. X-ray contrast agents).

Where previously disclosed reagents such as methylene diacrylate or dimethacrylate are used in accordance with this aspect of the invention, the reaction conditions will be chosen so as to ensure biodegradability of the product, e.g. by using a non-free radical mechanism such as Michael addition of nucleophiles, for example with reactive substrate groups such as hydroxyl groups, or by effecting copolymerisation with substrates such as acrylonitrile which may polymerise by non-radical mechanisms. Free radical polymerisations should desirably be effected in such a way as to avoid formation of excessively long or tightly crosslinked carbon chains, e.g. so as to produce polymers having a molecular weight not exceeding 40,000.

The following non-limitative Examples serve to illustrate the invention.

### EXAMPLE 1

### Methylene dimethacrylate

A solution of potassium hydroxide (1.00 M, 40.00 ml) is added to methacrylic acid (3.44 g, 40.00 mmol) at 0°C and the solution freeze dried for 16 hours. Dry dimethylformamide (230 ml) is added and the suspension heated to 60°C under a dry nitrogen atmosphere. Diiodomethane (1.61 ml, 20.00 mmol) is added in two portions during 10 min. and the reaction mixture left for 4 days at 60°C. The solvent is removed under reduced pressure (0.05 mm Hg), before diethyl ether (140 ml), saturated aqueous sodium hydrogen carbonate (50 ml) and water (50 ml) are added. The aqueous layer is extracted with diethyl ether (6 x 60 ml) and the combined ether extracts washed with water (4 x 50 ml), dried (MgSO₄), and evaporated to give 2.63 g (72%) of the title compound. ¹H NMR (60 MHz, CDCl₃): δ 1.97 (2 x CH₃, m), 5.63 (2 x H-C=, m), 5.88 (CH₂, s) 6.18 (2 x H-C=, m). IR (film, cm⁻¹): 2987 (w), 2962 (w), 2930 (w), 1732 (str), 1638 (w), 1454 (w), 1315 (w), 1295 (w), 1158 (w), 1100 (str), 1012 (m), 989 (m). This product may be used in accordance with the invention, for example to crosslink acrylamide polymers.

### EXAMPLE 2

### Methylene diacrylate

A solution of potassium hydroxide (1.00 M, 40.00 ml) is added to acrylic acid (2.88 g, 40.00 mmol) at 0°C and the solution freeze dried for 16 hours. Dry dimethylformamide (200 ml) is added and the suspension heated to 60°C under a dry nitrogen atmosphere. Diiodomethane (1.61 ml, 20.00 mmol) is added in two portions during 10 min. and the reaction mixture left for 4 days at 60°C. The solvent is removed under reduced pressure (0.05 mm Hg), before diethyl ether (140 ml), saturated aqueous sodium hydrogen carbonate (50 ml) and water (50 ml) are added. The aqueous layer is extracted with diethyl ether (6 x 60 ml) and the combined ether extracts washed with water (4 x 50 ml), dried (MgSO₄), and evaporated to give 1.06 g (34%) of the title compound. ¹H NMR (60 MHz, CDCl₃) : δ 5.81-6.61 (2 x CH₂ = CH-, m), 5.84 (CH₂, s). This product may be used in accordance with the invention, for example to crosslink acrylic acid and methyl acrylate polymers.

### EXAMPLE 3

### Chloromethyl (2-methacryloyloxy)ethyl carbonate

Pyridine (0.89 ml, 11.00 mmol) is added dropwise to a solution of chloromethyl chloroformate (0.89 ml, 11.00 mmol) and 2-hydroxyethyl methacrylate (1.22 ml, 10.00 mmol) in dichloromethane (12 ml) at 0°C under a dry nitrogen atmosphere. After 21 hours at 20°C the reaction mixture is washed with hydrochloric acid (1.00 M, 10 ml), saturated aqueous sodium hydrogen carbonate (10 ml) and water (10 ml). The organic phase is dried (MgSO₄) and the solvent evaporated under reduced pressure (10 mm Hg) to give 1.97g (88%) of the title compound. ¹H NMR (60 MHz, CDCl₃): δ 1.88 (CH₃, d, J=2 Hz), 4.35 (O-CH₂-CH₂-O, m), 5.47 (H-C=, m), 5.63 (CH₂-Cl, s), 6.00 (H-C=, m).

### EXAMPLE 4

### (2-Methacryloyloxy)ethyl methacryloyloxymethyl carbonate

A solution of potassium hydroxide (1.00 M, 5.00 ml) is added to methacrylic acid (0.43 g, 5.00 mmol) at 0°C and the solution freeze dried during 16 hours. Dry dimethylformamide (50 ml) is added and to the resulting suspension is added chloromethyl (2-methacryloyloxy)ethyl carbonate (1.11 g, 5.00 mmol). 18-Crown-6 (0.066 g, 0.25 mmol) is added as a catalyst and the reaction left under a dry nitrogen atmosphere. After 24 hours at 20°C and 6 days at 4°C the solvent is removed under reduced pressure (0.05 mm Hg) and diethyl ether (30 ml) and water (20 ml) added. The aqueous layer is extracted with diethyl ether (3 x 20 ml) and the combined ether extracts washed with water (20 ml), dried (MgSO₄) and evaporated to give 1.26 g (93%) of the title compound. ¹H NMR (60 MHz, CDCl₃): δ 1.97 (2 x CH₃, m), 4.38 (O-CH₂-CH₂-O, m), 5.53 (2 x H-C=, m), 5.77 (CH₂, s), 6.07 (2 x H-C=, m).

### EXAMPLE 5

### Ethylene bis(chloromethyl carbonate)

Pyridine (0.89 ml, 11.00 mmol) is added dropwise to a solution of chloromethyl chloroformate (1.32 ml, 14.83 mmol) and ethylene glycol (0.28 ml, 5.00 mmol) in dichloromethane (10 ml) at 7°C with good stirring under a dry N₂ atmosphere. After 15 min. at 7°C and 6 hours at 20°C the reaction mixture is transferred to a separating funnel with the aid of dichloromethane (10 ml). The reaction mixture is washed with hydrochloric acid (1.00 M, 10 ml), saturated aqueous sodium hydrogen carbonate (10 ml) and water (10 ml). The organic phase is dried (MgSO₄) and the solvent evaporated under reduced pressure to give 1.12g (90%) of the title product. ¹H NMR (300 MHz, CDCl₃): δ 4.48 (s, O-CH₂CH₂-O), 5.75 (s, 2 x Cl-CH₂-O). ¹³C NMR (75 MHz, CDCl₃): δ 65.8 -(O-CH₂CH₂-O) , 72.2 (2 x Cl-CH₂-O), 153.0 (2 x C=O).

### EXAMPLE 6

### Bis(2-chloromethoxycarbonylozyethyl)ether

Pyridine (0.89 ml, 11.00 mmol) is added dropwise to a solution of chloromethyl chloroformate (1.32 ml, 14.83 mmol) and diethylene glycol (0.47 ml, 5.00 mmol) in dichloromethane (10 ml) at 7°C with good stirring under a dry N₂ atmosphere. After 10 min. at 7°C and 6 hours at 20°C the reaction mixture is transferred to a separating funnel with the aid of dichloromethane (10 ml). The reaction mixture is washed with hydrochloric acid (1.00 M, 10 ml), saturated aqueous sodium hydrogen carbonate (10 ml) and water (10 ml). The organic phase is dried (MgSO₄) and the solvent evaporated under reduced pressure (10 mm Hg) to give 1.26 g (86%) title product. ¹H NMR (300 MHz, CDCl₃): δ 3.72 (m, 2 x CH₂-O), 4.34 (m, 2 x CH₂-O-C=O), 5.71 (s, 2 x Cl-CH₂-O). ¹³C NMR (75 MHz, CDCl₃): δ 67.6 (2 x CH₂-O), 68.5 (2 x CH₂-O-C=O), 72.1 (2 x Cl-CH₂-O), 153.2 (2 x C=O).

### EXAMPLE 7

### 1-Chloroethyl 2-methacryloyloxyethyl carbonate

Pyridine (0.89 ml, 11.00 mmol) is added dropwise to a solution of 1-chloroethyl chloroformate (1.20 ml, 11.00 mmol) and 2-hydroxyethyl methacrylate (1.22 ml, 10.00 mmol) in dichloromethane (12 ml) at 3°C under a dry N₂ atmosphere. After 15 min. at 3°C and 17 hours at 20°C the reaction mixture is transferred to a separating funnel with the aid of dichloromethane (10 ml). The reaction mixture is washed with hydrochloric acid (1.00 M, 10 ml), saturated aqueous sodium hydrogen carbonate (10 ml) and water (2 x 10 ml). The organic phase is dried (MgSO₄) and the solvent evaporated under reduced pressure to give 1.76g (74%) of the title product. ¹H NMR (60 MHz, CDCl₃): δ 1.85 (3 H, d, J=6 Hz, CH₃-CH), 1.96 (3 H,d, J=2 Hz, CH₃-C=), 5.55 (1 H, m, CH=), 6.10 (1 H, m, CH=), 6.38 (1 H, k, J=6 Hz, CH-CH₃).

### EXAMPLE 8

### Chloromethyl 4-acryloyloxybutyl carbonate

Pyridine (0.89 ml, 11.00 mmol) is added dropwise to a solution of chloromethyl chloroformate (0.98 ml, 11.00 mmol) and 4-hydroxybutyl acrylate (1.38 ml, 10.00 mmol) in dichloromethane (12 ml) at 3°C under a dry N₂ atmosphere. After 15 min. at 3°C and 17 hours at 20°C the reaction mixture is transferred to a separating funnel with the aid of dichloromethane (10 ml). The reaction mixture is washed with hydrochloric acid (1.00 M, 10 ml), saturated aqueous sodium hydrogen carbonate (10 ml) and water (2 x 10 ml). The organic phase is dried (MgSO₄) and the solvent evaporated under reduced pressure to give 1.76g (74%) of the title product. ¹H NMR (60 MHz, CDCl₃): δ 1.82 (4 H, m,CH₂-CH₂), 4.27 (4 H, m, 2 x CH₂-O), 5.77 (2 H, s, Cl-CH₂-O), 5.8-6.7 (3 H, m, CH=CH₂).

### EXAMPLE 9

### 1-Chloroethyl 4-acryloyloxybutyl carbonate

Pyridine (0.89 ml, 11.00 mmol) is added dropwise to a solution of 1-chloroethyl chloroformate (1.20 ml, 11.00 mmol) and 4-hydroxybutyl acrylate (1.38 ml, 10.00 mmol) in dichloromethane (12 ml) at 3°C under a dry N₂ atmosphere. After 15 min. at 3°C and 17 hours at 20°C the reaction mixture is transferred td a separating funnel with the aid of dichloromethane (10 ml). The reaction mixture is washed with hydrochloric acid (1.00 M, 10 ml), saturated aqueous sodium hydrogen carbonate (10 ml) and water (2 x 10 ml). The organic phase is dried (MgSO₄) and the solvent evaporated under reduced pressure to give 2.26g (90%) of the title product. ¹H NMR (60 MHz, CDCl₃): δ 1.80 (4 H, m, CH₂-CH₂), 1.86 (3 H, d, J=5 Hz, CH₃), 4.24 (4 H, m, 2 x CH₂-O), 5.7-6.6 (4 H, m, CH=CH₂ and CH).

### EXAMPLE 10

### 1-Methacryloyloxyethyl 2-methacryloyloxyethyl carbonate

1-Chloroethyl 2-methacryloyloxyethyl carbonate (1.183g, 5.00 mmol) prepared as described in Example 7 is added to a suspension of freeze dried potassium methacrylate (0.683 g, 5.50 mmol) and 18-crown-6 (0.066 g, 0.25 mmol) in dimethylformamide (50 ml) under a dry N₂ atmosphere. After 5 days at 20°C the solvent is removed under reduced pressure and the residue dissolved by adding dichloromethane (60 ml) and water (30 ml). After separating the phases the aqueous layer is extracted with dichloromethane (3 x 30 ml) and the combined organic phase washed with saturated aqueous sodium hydrogen carbonate (50 ml). The organic phase is dried (mgSO₄) and the solvent removed under reduced pressure to give 1.10g (77%) of the title product. ¹H NMR (60 MHz, CDCl₃): δ 1.63 (3 H, d, J=5 Hz, CH₃-CH), 1.98 (6 H, s, 2 x CH₃), 4.42 (4 H, s, 0-CH₂-CH₂-O), 5.62 (2 H, m, CH=), 6.15 (2 H, m, CH=), 6.84 (1 H, k, J=5 Hz, CH-CH₃).

### EXAMPLE 11

### Acryloyloxymethyl 4-acryloyloxybutyl carbonate

Chloromethyl 4-acryloyloxybutyl carbonate (1.183g, 5.00 mmol) prepared as described in Example 8 is added to a suspension of freeze dried potassium acrylate (0.606 g, 5.50 mmol) and 18-crown-6 (0.066 g, 0.25 mmol) in dimethylformamide (50 ml) under a dry N₂ atmosphere. After 5 days at 20°C the solvent is removed under reduced pressure and the residue dissolved by adding dichloromethane (60 ml) and water (30 ml). After separating the phases the aqueous layer is extracted with dichloromethane (3 x 30 ml) and the combined organic phase washed with saturated aqueous sodium hydrogen carbonate (50 ml). The organic phase is dried (MgSO4) and the solvent removed under reduced pressure to give 1.24g (91%) of the title product. ¹H NMR (60 MHz, CDCl₃): δ 1.82 (4 H, m, CH₂-CH₂), 4.23 (4 H, m, 2 x CH₂-O), 5.88 (2 H, s, O-CH₂-O), 5.7-6.8 (6 H, 2 x CH=CH₂).

### EXAMPLE 12

### 1-Acryloyloxyethyl 4-acryloyloxybutyl carbonate

1-Chloroethyl 4-acryloyloxybutyl carbonate (1.253g, 5.00 mmol) prepared as described in Example 9 is added to a suspension of freeze dried potassium acrylate (0.606 g, 5.50 mmol) and 18-crown-6 (0.066 g, 0.25 mmol) in dimethylformamide (50 ml) under a dry N₂ atmosphere. After 5 days at 20°C the solvent is removed under reduced pressure and the residue dissolved by adding dichloromethane (60 ml) and water (30 ml). After separating the phases the aqueous layer is extracted with dichloromethane (3 x 30 ml) and the combined organic phase washed with saturated aqueous sodium hydrogen carbonate (50 ml). The organic phase is dried (MgSO₄) and the solvent removed under reduced pressure to give 1.28g (89%) of the title product. ¹H NMR (60 MHz, CDCl₃): δ 1.58 (3 H, d, J=5 Hz, CH₃-CH), 1.80 (4 H, m, CH₂-CH₂), 4.24 (4 H, m, 2 x CH₂-O), 5.7-6.7 (6 H, m, 2 x CH=CH₂), 6.87 (1 H, k, J=5 Hz, CH-CH₃).

### EXAMPLE 13

### Methylene bis(p-vinylbenzoate)

Diiodomethane (0.20 ml, 2.50 mmol) is added to a solution of freeze dried potassium p-vinylbenzoate (0.931 g, 5.00 mmol), 18-crown-6 (0.040 g, 0.25 mmol) and hydroquinone (0.011 g, 0.10 mmol) in dimethylformamide (35 ml) under a dry N₂ atmosphere and the reaction mixture left for 2.5 days at 60°C. The solvent is removed under reduced pressure and the residue dissolved by adding diethyl ether (20 ml), saturated aqueous sodium hydrogen carbonate (5 ml) and water (10 ml). After separating the phases the aqueous layer is extracted with diethyl ether (6 x 10 ml) and the combined organic phase washed with water (5 x 10 ml). The organic phase is dried (MgSO₄) and the solvent removed under reduced pressure to give 0.64g (83%) of the title product. ¹H NMR (300 MHz, CDCl 3): δ 5.39 (2 H, d, J=10 Hz, 2 x CH=), 5.86 (2 H, d, J=17.6 Hz, 2 x CH=), 6.24 (2 H, s, O-CH2-O), 6.73 (2 H, dd, J=11.0, 17.6, 2 x CH=), 7.45 (4 H, 2 x d, J=6.8 Hz, Ar), 8.04 (2 H, d, J=6.6 Hz, Ar), 8.05 (2 H, d, J=6.6 Hz, Ar). ¹³C NMR (75 MHz, CDCl₃): δ 79.8 (O-CH₂-O), 116.8 (2 x CH=), 126.0, 130.2 (C₂,C₂',C₃, C₃'), 127.8, 142.5 (C₁,C₁',C₄,C₄'), 135.7 (2 x CH=), 164.9 (2 x C=O).

### EXAMPLE 14

### Methylene bis(p-bromobenzoate)

Diiodomethane (0.60 ml, 7.50 mmol) is added to a solution of freeze dried potassium p-bromobenzoate (3.587 g, 15.00 mmol) and 18-crown-6 (0.198 g, 0.75 mmol) in dimethylformamide (100 ml) under a dry N₂ atmosphere and the reaction mixture left for 4 days at 60°C. The solvent is removed under reduced pressure and the residue dissolved by adding dichloromethane (60 ml) and water (30 ml). After separating the phases the aqueous layer is extracted with dichloromethane (3 x 30 ml) and the combined organic phase washed with saturated aqueous sodium hydrogen carbonate (50 ml). The organic phase is dried (MgSO₄) and the solvent removed under reduced pressure to give 2.62g (84%) of the title product. ¹H NMR (60 MHz, CDCl₃): δ 6.29 (2 H, s, O-CH₂-O), 7.63 (4 H, d, J=9 Hz, Ar), 8.00 (4 H, d, J=9 Hz, Ar).

### EXAMPLE 15

### Methylene bis(p-hydroxybenzoate)

Diiodomethane (0.40 ml, 5.00 mmol) is added to a solution of freeze dried potassium p-hydroxybenzoate (1.762 g, 10.00 mmol) in dimethylformamide (60 ml) under a dry N₂ atmosphere and the reaction mixture left for 4 days at 60°C. The solvent is removed under reduced pressure and the residue dissolved by adding dichloromethane (60 ml) and water (30 ml). After separating the phases the aqueous layer is extracted with dichloromethane (3 x 30 ml) and the combined organic phase washed with brine (50 ml). The organic phase is dried (MgSO₄) and the solvent removed under reduced pressure to give 0.94g (65%) of the title product. ¹H NMR (60 MHz, CDCl₃/CD₃OD 1:2): δ 4.92 (2 H, s, 2 x OH), 6.18 (2 H, s, O-CH₂-O), 6.88 (4 H, d, J=9 Hz, Ar), 7.96 (4 H, d, J=9 Hz, Ar).

### EXAMPLE 16

### Methylene bis[p-(hydroxymethylethynyl)benzoate]

Bis (triphenylphosphine)palladium dichloride (17.0 mg, 0.02 mmol) and cuprous iodide (2.0 mg, 0.01mmol) are added to a suspension of methylene bis (p-bromobenzoate) (0.500 g, 1.21 mmol) prepared as described in Example 14 and propargyl alcohol (0.16 ml, 2.66 mmol) in triethylamine (10 ml) with good stirring, at 20°C, under a dry N₂ atmosphere. After 10 days at 20°C, the triethylamine is removed under reduced pressure, water (20 ml) is added and the mixture is extracted with dichloromethane (3 x 15 ml). The dichloromethane phases are washed with hydrochloric acid (0.5 M, 10 ml), dried (MgSO₄) and the dichloromethane removed under reduced pressure to give 0.37 g (85%) of the crude product. ¹H NMR (60 MHz, CDCl₃): δ 3.67 (2 H, s, OH), 4.47 (4 H, s, CH₂-O), 6.18 (2 H, s, O-CH₂-O), 7.2-7.5 (4 H, Ar), 7.8-8.0 (4 H, Ar).

### EXAMPLE 17

### Adipic acid bis (1-chloroethyl ester)

Anhydrous zinc chloride (10.0 mg, 0.07 mmol) is added to adipoyl chloride (2.92 ml, 20.00 mmol) at 20°C, under a dry N₂ atmosphere. Acetaldehyde (2.26 g, 40.00 mmol) is added dropwise to the reaction mixture at -5°C. The reaction temperature is kept between -5°C and 0°C and dichloromethane (20 ml) is added. The zinc chloride catalyst is removed by passing the reaction mixture through a chromatography column containing aluminium oxide (Fluka 06290, type 5016 A basic, 20 g) at 5°C using dichloromethane as the solvent. The solvent is removed under reduced pressure to give 3.64 g (67%) of the crude product. ¹H NMR (60 MHz, CDCl₃): δ 1.5-1.9 (4 H, m, CH₂-CH₂), 1.77 (6 H, d, J=6 Hz, 2 x CH₃) , 2.1-2.5 (4 H, m, 2 x CH₂-O), 6.49 (2 H, k, J=6 Hz, 2 x Cl-CH-O).

### EXAMPLE 18

### Methylene bis [p-(2,3-epoxy-1-propyloxy)benzoate]

Potassium tert.butoxide (1.347 g, 12.00 mmol) is added to a solution of methylene di(p-hydroxybenzoate) (1.728 g, 6.00 mmol) prepared as described in Example 15 in DMF (75 ml), under a dry N₂ atmosphere. Epichlorohydrin (2.22 g, 24.00 mmol) is added and after 24 hours at 20°C the solvent is removed under reduced pressure. The residue is dissolved by adding dichloromethane (75 ml) and water (30 ml) and adjusting the pH to neutral using hydrochloric acid (1 M). After separating the phases the dichloromethane layer is washed with water (3 x 30 ml). The organic phase is dried (MgSO₄) and the solvent removed under reduced pressure to give 1.22 g (51%) product as a colourless oil. ¹H NMR (60 MHz, CDCl₃): δ 2.8 (4 H, m, 2 x epoxy-CH₂), 3.3 (2 H, m, 2 x epoxy-CH), 4.05 (2 H, dd, J=22, 11 Hz, 2 x O-CH-H), 4.12 (2 H, dd, J=22, 11 Hz, 2 x O-CH-H), 6.14 (2 H, s, O-CH₂-O), 6.9 (4 H, m, 2 x Ar), 7.9 (4 H, m, 2 x Ar).

### EXAMPLE 19

### Methylene bis(3,3-dimethoxypropionate)

Cesium 3,3-dimethoxypropionate (19.95 g, 75 mmol) is added to dry DMF (1000 ml). Diiodomethane (10.04 g, 37.5 mmol) is added to the suspension and the reaction mixture is stirred for 2 days at 60°C under a dry N₂ atmosphere. DMF is removed under reduced pressure (0.01 mmHg). Diethyl ether (500 ml) is added to the residue, which is then washed with saturated aqueous sodium hydrogen carbonate (250 ml). The aqueous layer is extracted with diethyl ether (5 x 75 ml). The combined ether extracts are washed with water (2 x 100 ml), dried (MgSO₄) and evaporated to give 7.1 g (72%) product. ¹H NMR (300 MHz, CDCl₃): δ 2.61 (CH₂, d), 3.26 (CH₃, s), 4.76 (CH,t), 5.70 (CH₂, s). ¹³C NMR (300 MHz, CDC₃): δ 38.52 (CH₂), 53.37 (CH₃O), 79.02 (OCH₂O), 168.32 (C=O) .

### EXAMPLE 20

### Methylene bis(3-methoxypropenoate)

Methylene bis(3,3-dimethoxypropionate) (14.01g, 50 mmol) prepared as described in Example 19 and a catalytic amount of p-toluene sulfonic acid is added to toluene (250 ml). The methanol is removed by warming the reaction under an N₂ atmosphere. When the reaction is complete the toluene is distilled off under reduced pressure. Diethyl ether (250 ml) is added and the mixture is washed with saturated aqueous sodium hydrogen carbonate (5x50 ml) and water (3x50 ml). The organic layer is dried (MgSO₄) before evaporation to give 8.52g (79%) product. ¹H NMR (300 MHz, CDCl₃): δ 3.65 (2 x CH₃, s), 5.2 (2 x CH, d), 5.8 (O-CH₂-O), 7.65 (2 x CH₂, d).

### EXAMPLE 21

### Methylene bis (10-undecenoate)

10-Undecylenic acid (12.75 g, 75 mmol) is dissolved in 100 ml water. Cesium carbonate (13.04 g, 40 mmol) is added to the mixture. The water is removed under reduced pressure and the salt dried for 2 hours in vacuo. The cesium salt is mixed with 150 ml DMF and diiodomethane is added to the solution. The reaction is stirred for 3 days at 60°C under an N₂ atmosphere. DMF is then removed under reduced pressure. The residue is purified through silica gel with hexane/ ethyl acetate (8:2) as eluant. The solvent is evaporated to give 7.18 g (54%) product. ¹H NMR (300 MHz, CDCl₃): δ 1.2-1.4 (10 x CH₂, m), 1.6 (2 x CH₂, m), 2.0 (2 x CH₂, m), 2.19 (2 x CH₂, t), 4.9 (2 x H₂ C=, m), 5.88 (O-CH₂-O, s), 5.9 (2 x HC=, m). ¹³C NMR (300 MHz, CDCl₃): δ 24.92-33.98 (8 x CH₂), 79.04 (O-CH₂-O), 114.18 (=CH₂), 139.11 (=CH), 172.48 (C=O).

### EXAMPLE 22

### Methylene bis(10-epoxyundecanoate)

Methylene bis(10-undecenoate) (8.8g, 25 mmol) prepared as described in Example 21 is added under an N₂ atmosphere to methylene chloride and cooled to O°C. Metachloroperbenzoic acid 55% (15.75g, 50 mmol) is added to methylene chloride (150 ml) and the organic layer is separated and dried (MgSO₄). The metachloroperbenzoic acid is then added dropwise to the diester. After completed addition the temperature is increased to 25°C. After 5 hours the reaction is complete. The mixture is washed with saturated aqueous sodium sulphite (75 ml) and saturated aqueous sodium hydrogen carbonate (2 x 75 ml). The organic layer is purified on neutral aluminium oxide. The solvent is removed under reduced pressure to yield 8.45g (82%) product. ¹H NMR (300 MHz, CDCl₃): δ 1.2-1.7(14 x CH₂, m), 2.35(2 x CH₂CO,t), 2.45 (2 x CH,q), 2.75 (2 x CH,q), 2.90 (2 x CH,m), 5.75 (0-CH₂-0). ¹³C NMR (300 MHz, CDCl₃): δ 24.58 (CH₂), 25.99 (CH₂), 28.94 (CH₂), 29.09 (CH₂), 29.32 (2 x CH₂), 32.45 (CH₂), 33.92 (CH₂), 47.06 (CH₂-0), 52.-36 (CH-0), 79.06 (0-CH₂-0), 172.2 (C=O).

### EXAMPLE 23

### Methylene bis(hydroxyacetate)

### (a) Methylene bis(benzyloxyacetate)

Benzyloxyacetic acid (49.8 g, 300 mmol) is dissolved in a 500 ml mixture of water and MeOH (60:40), and cesium carbonate (48.9 g, 150 mmol) is added to the solution. The solvent is evaporated under reduced pressure and residual water is removed azeotropically with benzene. The salt is dissolved in 1500 ml DMF and diiodomethane (40.2 g, 150 mmol) is added to the solution. The reaction mixture is stirred for 3 days at 60°C under an N₂ atmosphere. The DMF is removed under reduced pressure and the residue is dissolved in ether (250 ml) and washed with saturated aqueous sodium hydrogen carbonate (250 ml) and water (3 x 75 ml) before drying (MgSO₄). The solvent is evaporated and the residue is purified through silica gel with hexane/ethyl acetate (7:3) as eluant to give 23.6 g (46%) product. ¹H NMR (300 MHz, CDCl₃): δ 4.1 (2 x CH₂, s), 4.6 (2 x CH₂, s), 5.9 (O-CH₂-O, s), 7.35 (2 x C₆ H₅, m).

### (b) Methylene bis(hydroxyacetate)

Methylene bis(benzyloxyacetate) (0.52 g, 1.5 mmol) and Pd/C (100 mg, 10%) are added to dry ethanol (100 ml). Hydrogen (1 atm) is introduced and the reaction is complete after 16 hours at room temperature, whereupon the reaction mixture is filtered and the solvent is evaporated under reduced pressure (0.01 mmHg) to yield 0.23 g (95%) product. ¹H NMR (200 MHz, MeOH): δ 4.2 (CH₂, s), 4.9 (OH), 5.9 (OCH₂0, s). The product may be used to form polyesters with di- or poly- acids and to form polyurethanes with isocyanates.

### EXAMPLE 24

### Methylene bis(16-hydroxyhexadecanoate)

### (a) 16-Triphenylmethoxyhexadecanoic acid

A solution of 16-hydroxyhexadecanoic acid (1.36 g, 5.00 mmol), triphenylmethyl chloride (1.53 g, 5.50 mmol), triethylamine (1.25 ml) and 4-dimethylaminopyridine (10.03 g, 0.25 mmol) is stirred overnight in dry dimethylformamide at ambient temperature under nitrogen. After 16 hours stirring, the brown cloudy solution is poured into ice-water and extracted with dichloromethane (5 X 50 ml). The organic phases are washed with saturated ammonium chloride solution (2 X 100 ml), water (2 X 100 ml) and dried over MgSO₄. The solvent is removed under reduced pressure and the product purified by flash chromatography on a silica column with dichloromethane/methanol (20:1) as eluant to yield the title compound as a yellow oil (0.41 g). ¹³C NMR (75 MHz, CDCl₃): δ 24.9, 25.7, 26.3, 29.2, 29.5, 29.6, 29.7, 30.0, 32.8, 34.1, 62.9, 63.7, 86.2, 144.5, 177.2. MS (CI): 515 (M + H)

### (b) 16-Triphenylmethoxyhexadecanoic acid cesium salt

Aqueous cesium carbonate (1M, 0.16 ml) is added dropwise to a solution of 16-triphenylmethoxyhexa-decanoic acid (0.16 g, 0.31 mmol) in tetrahydrofuran (10 ml) until the pH reaches approximately 8, whereupon the solvent is removed under reduced pressure and the residue dried under vacuum for 2 hours. The oily semicrystalline residue is dispersed in dry dimethylformamide (10 ml) and evaporated to dryness in vacuo. The crystalline product is used without further characterization.

### (c) Methylene bis(16-triphenylmethoxyhexadecanoate)

Diiodomethane (0.04 g, 0.16 mmol) is added to a suspension of 16-triphenylmethoxyhexadecanoic acid cesium salt (0.31 mmol) in dry dimethylformamide (10 ml). The reaction mixture is heated at 60 °C for 2 days under nitrogen. The solvent is removed in vacuo, and the product purified by flash chromatography on a 2 x 5 cm silica column with chloroform as eluant to yield the title compound as a brown oil (0.10 g). ¹³C NMR (75 MHz, CDCl₃): δ 24.6, 26.3, 29.0, 29.2, 29.4, 29.5, 29.6, 29.7, 30.0, 34.0, 63.7, 79.0, 86.2, 126.7, 127.2, 127.6, 127.9, 128.7, 144.5, 172.5.

### (d) Methylene bis(16-hydroxyhexadecanoate)

Methylene bis(16-triphenylmethoxyhexadecanoate) (0.07g, 0.07 mmol) is dissolved in glacial acetic acid (8 ml) and heated at 55°C. The reaction is monitored by TLC. After 10 hours the reaction mixture is poured onto ice, and the crude product is filtered, washed with aqueous sodium bicarbonate and water, and dried under reduced pressure. The product is purified by flash chromatography on a silica column with chloroform/ methanol (20:1) as eluant to yield the title compound as a white solid. ¹H-NMR (300 MHz, CDCl₃) : δ 1.2-1.4(m, 44H), 1.5-1.6(m, 8H), 2.35(t, 4H), 3.64(t, 4H), 5.75(s, 2H).

### EXAMPLE 25

### Methylene bis(hydrogen azelate)

### (a) Benzyl hydrogen azelate

Toluene-4-sulfonic acid monohydrate (0.71 g, 3.72 mmol) is added to a suspension of azelaic acid (25.0 g, 132.82 mmol) in benzene (550 ml). The mixture is heated to 80°C, whereafter benzyl alcohol (14.36 g, 132.82 mmol) in benzene (50 ml) is added dropwise to the resulting solution. The reaction mixture is refluxed overnight and water is removed azeotropically with a Dean Stark trap. The reaction mixture is allowed to cool, the white precipitate which forms is removed by filtration and the filtrate is concentrated to a brownish oil under reduced pressure. The crude product (33.97 g) is dissolved in dichloromethane (50 ml) and purified by flash chromatography on a 5.5 x 15 cm silica column with dichloromethane/methanol (20:1) as eluant. The product, a yellow oil, is dried under vacuum. The oil crystallizes after a few hours at room temperature. Yield: 12.8 g (35%). ¹³C NMR (75 MHz, CDCl₃): δ 24.5, 24.8, 28.8, 34.0, 34.2, 66.1, 128.2, 128.5, 136.1, 173.6, 180.0.

### (b) Cesium benzyl azelate

Aqueous cesium carbonate (1M, 6.3 ml) is added dropwise to a solution of benzyl hydrogen azelate (3.00 g, 10.77 mmol) in 75 ml water/methanol (1:15) until the pH reaches approximately 7, whereupon the solvent is removed under reduced pressure and the residue dried under vacuum overnight. The oily, yellowish semicrystalline residue is dispersed in dry dimethylformamide (50 ml) and evaporated to dryness in vacuo. This procedure is repeated twice, yielding an off-white crystalline product. The product is used without further characterization.

### (c) Methylene bis(benzyl azelate)

Diiodomethane (1.44 g, 5.37 mmol) is added to a suspension of cesium benzyl azelate (4.41 g, 10.77 mmol) in dry dimethylformamide (75 ml) under nitrogen. The reaction mixture is heated at 60°C for 2 days, whereafter the solvent is removed under reduced pressure and the residue is transferred to an extraction funnel with ethyl acetate (150 ml) and water (75 ml). The organic phase is extracted with water (3x50 ml), dried over MgSO₄ and concentrated to a yellow oil in vacuo. Yield: 2.86 g (95.6%). ¹³C NMR (75 MHz, CDCl₃): δ 24.4, 24.8, 28.7, 28.8, 28.9, 33.8, 34.2, 66.0, 79.0, 128.1, 128.5, 136.1, 172.3, 173.5.

### (d) Methylene bis(hydrogen azelate)

Methylene bis(benzyl azelate) (10 g, 17.58 mmol) is dissolved in glacial acetic acid (250 ml). 10% Pd/C (2.0 g) is added, and hydrogen gas is bubbled through the solution for 2 hours. The reaction is monitored by TLC. The catalyst is removed by filtration and the solvent is removed under reduced pressure. The crude product is dissolved in diethyl ether and petroleum ether is added. An oil precipitates, which crystallizes after 1 hour. The mixture is left in a refrigerator overnight before the crystals are collected by filtration and dried under vacuum, to yield the title compound. Yield: 5.33 g (78%). ¹³C NMR (75 MHz, CDCl₃): δ 24.5, 24.6, 28.7, 28.8, 33.9, 79.1, 172.5, 180.0. mp: 57-60°C.

### EXAMPLE 26

### Methylene bis(hydrogen tetracosanedioate)

### (a) Benzyl hydrogen tetracosanedioate

Toluene-4-sulfonic acid monohydrate (0.05 g, 0.28 mmol) is added to a suspension of tetracosanedioic acid (5.0 g, 80%, 10.03 mmol) in benzene (180 ml). The mixture is heated to 80°C, whereafter benzyl alcohol (1.08 g, 10.03 mmol) in benzene (10 ml) is added dropwise to the resulting solution. The reaction mixture is refluxed for 20 hours and water is removed azeotropically with a Dean Stark trap. The solvent is removed under reduced pressure and the residue washed with petroleum ether. The product is dissolved in refluxing diethyl ether and purified by flash chromatography on a silica column with methylene chloride/methanol (20:1) as eluant to yield the title compound as a white crystalline solid. ¹³C NMR (75 MHz, CDCl₃): δ 24.0, 28.5, 29.7, 30.9, 34.4, 66.2, 128.2, 128.5, 136.0, 174.1, 176.9.

### (b) Methylene bis(hydrogen tetracosanedioate)

The product from (a) above is reacted in similar manner to that described in Example 25 (b)-(d) to yield the title compound.

### EXAMPLE 27

### Methylene bis(4-pentenoate)

4-Pentenoic acid (10g, 100 mmol), diiodomethane (13.4 g, 50 mmol) and 1,8-diazabicyclo[5.4.0]undec-7-ene (15.25 g, 100 mmol) are dissolved in acetonitrile (150 ml). The solution is refluxed under nitrogen for 3 hrs, whereafter acetonitrile is removed under reduced pressure. The residue is dissolved in water (75 ml) and extracted with diethyl ether (3x 100 ml). The combined ether extracts are washed with saturated aqueous sodium carbonate (50 ml), dried (MgSO₄) and evaporated to give 8.39 g (79%) product. ¹³C NMR (75 MHz, CDCl₃): δ 28.48 (2xCH₂), 33.20 (2xCH₂), 79.11 (O-CH₂-O), 115.80 (2xH₂C=), 136.18 (2x = CH-), 171.68 (2x C=O).

### EXAMPLE 28

### Methylene bis(4-epoxypentanoate)

Metachloroperbenzoic acid (15.68 g, 55%, 50 mmol) is dissolved in methylene chloride (200 ml). Water is separated and the organic layer is dried (MgSO₄). The resulting metachloroperbenzoic acid solution is added dropwise to methylene bis(4-pentenoate) (4.10 g, 19 mmol) dissolved in methylene chloride (50 ml). The mixture is stirred at ambient temperature under nitrogen for 12 hrs, whereafter the reaction mixture is washed with saturated aqueous sodium bicarbonate solution (50 ml), water (50 ml), dried (MgSO₄) and evaporated to give 3.61g (78%) of the title compound as a crystalline product. ¹H NMR (300 MHz, CDCl₃): δ 1.70-1.85 (2xCH,m), 1.95-2.10 (2x CH,m), 2.50-2.55 (2xCH, 2xCH₂,m), 2.75 (2xCH,t) , 3.0 (2xCH,m), 5.8 (O-CH₂-O, s). ¹³C NMR (75 MHz, CDCl₃): δ 27 (2xCH₂), 30 (2xCH₂), 47 (2xCH₂), 51 (2xCH), 79.8 (O-CH₂-O), 171.8 (2xC=O).

### EXAMPLE 29

### Methylene bis(2-butenoate)

Vinylacetic acid (4.3 g, 50 mmol) is added to an aqueous cesium carbonate solution (50 ml). The mixture is stirred for 5 min. and then evaporated, and the residue is dried under vacuum for 2 hrs. The resulting cesium salt and diiodomethane are added to dimethylformamide (200 ml) and the mixture is stirred for 24 hrs. at 50°C under nitrogen, whereafter the dimethylformamide is removed under reduced pressure. The residue is dissolved in diethyl ether (100 ml) and washed with saturated aqueous sodium bicarbonate (25 ml) and water (25 ml). The organic layer is dried (MgSO₄) and evaporated to give 1.32 g (29%) product. ¹H NMR (300 MHz, CDCl₃): δ 1.9 (2xCH₂,m), 5.8-5.9 (2xCH,m), 5.9 (OCH₂O,s), 7.0-7.1 (2xCH,m).

### EXAMPLE 30

### Methylene bis(chloroacetate)

Chloroacetic anhydride (12.75 g, 75 mmol), paraformaldehyde (2.25 g, 75 mmol) and conc. sulfuric acid (15 drops) are added to methylene chloride (15 ml). The mixture is stirred for 24 hrs. at 50°C under nitrogen, whereafter the reaction mixture is extracted with saturated aqueous potassium carbonate until carbon dioxide emission ends. The organic layer is dried (MgSO₄), evaporated to dryness and the residue is distilled (80°C, 0.15 mmHg) to yield 10.2 g (57%) product. ¹H NMR (200 MHz, CDCl₃): δ 4.1 (2xCH₂Cl,s), 5.9 (CH₂,s). ¹³C NMR (200 MHz, CDCl₃): δ 41.1 (CH₂Cl), 81.4 (O-CH₂-O) , 166.4 (CO).

### EXAMPLE 31

### Methylene bis(4-oxopentanoate)

4-Oxopentanoic acid (11.6 g, 100 mmol) is dissolved in acetonitrile (70 ml), and 1,8-diazabicyclo[5.4.0]undec-7-ene (15.25 g, 100 mmol) diluted with acetonitrile (30 ml) is added. Diiodomethane (13.4 g, 50 mmol) is added in one batch, and the reaction mixture is refluxed under a nitrogen atmosphere. After 2 hours, gas chromatography indicates full consumption of diiodomethane. The solvent is removed in vacuo and the residual brown oil is transferred to a separation funnel with ethyl acetate (200 ml) and water (75 ml). The organic phase is washed with 1M sodium bicarbonate (25 ml) and water (3 x 25 ml), dried over MgSO₄, and the solvent is removed in vacuo to yield the title compound (10 g). 1H NMR: δ 2.19 (2 x CH₃, s), 2.760-2.804 (2 x CH₂, t), 2.600-2.645 (2 x CH₂, t), 5.735 (CH₂ bridge, s).

### EXAMPLE 32

### Methylene bis(hydrogen galutarate)

### (a) Benzyl hvdrogen glutarate

A suspension of glutaric anhydride (50 g, 430 mmol) in benzyl alcohol (54 g, 500 mmol) is heated at 105°C overnight, whereafter gas chromatography indicates full consumption of the anhydride. Purification of a 1.3g sample by flash chromatography on a 2.5 X 15 cm silica column with chloroform and methanol/chloroform (1:10) as eluants yields title compound (1.1 g). ¹H NMR: δ 1.945-1.993 (CH₂, m), 2.397-2.470 (2x CH₂, m), 5.117 (CH₂, s), 7.332-7.357 (C₆H₅, m). The remaining crude product is purified by short path distillation; the main fraction is collected at 150 - 160°C/0.04 mmHg. Yield: 90 g.

### (b) Cesium benzyl glutarate

Crude benzyl hydrogen glutarate (25 g, 100 mmol) is stirred in water (100 ml) to form a slurry. An aqueous solution of 1 M cesium carbonate is added until the pH reaches 7 (52 ml is consumed). The homogeneous reaction mixture is diluted with water (150 ml), and extracted with chloroform (2 x 50 ml) to remove nonpolar impurities from the crude starting material. Water is removed in vacuo, and the oily, grayish semicrystalline residue is slurried in dimethylformamide (200 ml), and evaporated to dryness in vacuo. This procedure is repeated twice, yielding an off-white crystalline product, which is used without further characterization.

### (c) Methylene bis(benzyl glutarate)

Cesium benzyl glutarate (100 mmol) is slurried in dimethylformamide (150 ml). Diiodomethane (13.4 g, 50 mmol) is added, and the reaction mixture is heated at 70°C overnight under a nitrogen atmosphere. The resulting reaction mixture is a dark, brownish slurry, which is rendered homogeneous by addition of water (50 ml). The solvent is removed in vacuo, and the residue is transferred to an extraction funnel with ethyl acetate (200 ml) and water (100 ml). The organic phase is extracted with water (2 x 50 ml), dried over MgSO₄, and concentrated in vacuo to a brownish oil (15.5 g). 0.5 g of this product is purified by flash chromatography on a 2.5 x 15 cm silica column with methylene chloride and methanol/chloroform (1:10) as eluants to yield the title compound. ¹H NMR (300 MHz, CDCl₃): δ 1.94-1.99 (2 x CH₂, q), 2.40-2.44 (4 x CH₂, t), 5.11 (2 x CH₂, s), 5.28 (CH₂ bridge, s), 7.33-7.35 (2 x C₆H₅, m). The main part of the product is used without further purification.

### (d) Methylene bis(hydrogen glutarate)

Crude methylene bis(benzyl glutarate) (10 g, 22 mmol) is dissolved in a mixture of acetic acid (50 ml) and tetrahydrofuran (25 ml). 10% Pd/C (1.5 g) is added, and hydrogen gas is bubbled through the solution for 3h. The reaction is monitored by TLC. The catalyst is removed by filtration and the solvent is removed in vacuo. The crude product is dissolved in diethyl ether and hexane is added. An oil precipitates. After a few hours in a refrigerator, the oil crystallizes. The crystals are collected by filtration and dried under vacuum. Yield: 5g (80%). ¹³C NMR (75 MHz, CDCl₃): 171.627 ppm (CO-bridge), and 179.198 ppm (CO-free acid).

### EXAMPLE 33

### Methylene bis(succinimidylazelate)

1-(3-Dimethylaminopropyl)-3-ethylcarbodiimide hydrochloride (1.49 g, 7.71 mmol) was added in portions to a stirred solution of methylene bis(hydrogen azelate) from Example 25 (1.00 g, 2.57 mmol) and N-hydroxysuccinimide (0.89 g, 7.71 mmol) in dry dimethylformamide at ambient temperature. After 20 hours stirring, the reaction mixture was poured into ice-water, whereupon the product precipitated as an oil. The colourless oil was dissolved in diethylether (50 ml), washed with water (3x10 ml) and dried over MgSO₄. The solvent was removed under reduced pressure and hexane (5 ml) was added to the oily product. After seven days storage at 4°C the oil had crystallized to a white, waxy solid. Yield: 1.50 g (69%). m.p.: 45-47°C. ¹³C NMR (75 MHz, CDCl₃) : δ 24.42, 24.46, 25.59, 28.48, 28.63, 30.85, 33.82, 79.61, 168.6, 169.30, 172.34.

### EXAMPLE 34

### Methylene bis(16-acryloyloxyhexadecanoate)

Triethylamine (0.29 g, 2.87 mmol) in dry toluene (2 ml) was added to a suspension of methylene bis(16-hydroxydecanoate) from Example 24 (0.20 g, 0.36 mmol) in dry toluene (5 ml). The mixture was heated to 50°C under nitrogen and acryloylchloride (0.26 g, 2.87 mmol) in dry toluene (3 ml) was then added dropwise. After 1 hour of stirring at 55°C the reaction mixture was cooled to room temperature, diluted with toluene (10 ml), washed with water (2x5 ml) and dried over MgSO₄. The solvent was evaporated under reduced pressure to give a yellow solid product. Yield: 0.2 g (92%). MS (CI): 665 (M + H). ¹³C NMR (75 MHz, CDCl₃): δ 24.62, 25.93, 28.62, 29.01, 29.24, 29.26, 29.45, 29.52, 29.58, 29.60, 29.64, 33.98, 64.72, 78.99, 128.64, 130.43, 166.33, 172.52.

### EXAMPLE 35

### Methylene bis(10-methyl-6,8-dioxa-5,7-dioxoundecanoate)

Methylene bis(hydrogen glutarate) (1 g, 3.6 mmol) is dissolved in 25 ml dry acetone. Triethylamine (1 ml, 7.2 mmol) is added, and the reaction mixture is cooled to 0°C. Isobutylchloroformate (0.99 ml, 7.2 mmol) is added. The cooling bath is removed after 1 hour and stirring is continued for 1 hour. The reaction mixture is filtered and the solvent is removed in vacuo. The product is characterised by NMR, and is used without further purification.

### EXAMPLE 36

### Methylene bis(4-fluorocarbonyl)butyrate

Methylene bis(hydrogen glutarate) (1 g, 3.6 mmol) is reacted with cyanuric fluoride as described by Olah et al., Synthesis (1973) 487-488. The product is characterised by NMR and used without further purification.

### EXAMPLE 37

### Methylene bis(10-oxodecanoate)

### a) Methylene bis(10,11-dihydroxyundekanoate)

N-Methylmorpholine-N-oxide (13.5 g, 11 mmol) and methylene bis(10-undecenoate) from Example 21 (19 g, 5 mmol) were dissolved in 400 ml of a mixture of tetrahydrofuran and water (3:1 v/v). A catalytic amount of osmium tetroxide was added, and the solution stired at ambient temperature for 20 hours. TLC indicated complete consumption of the starting material. Excess sodium hydrogen sulphite and sodium chloride were then added to the reaction mixture. The product was extracted from the resulting mixture with ethyl acetate (400 ml) and the water phase was washed with ethyl acetate (3 x 50 ml). The combined organic phases were dried and evaporated, and the product recrystallised from tetrahydrofuran to yield 14.5g (68%) of the product as a white solid. ¹³C NMR (45 MHz) CD₃OD:δ 24.6-34.0 (16 x CH₂), 66.6 (2 X CH₂OH), 72.3 (2 X CHOH) 79.2 (O-CH₂-O), 174.0 (2 X C=O).

### b) Methylene bis(10-oxodecanoate)

Methylene bis(10,11-dihydroxyundecanoate) (2.24 g, 5 mmol) was dissolved in 150 ml tetrahydrofuran. Sodium metaperiodate (2.06 g, 10 mmol) was dissolved in 150 ml water and added dropwise to the tetrahydrofuran solution. TLC indicated full consumption of the diol after 60 minutes, whereupon sodium chloride was added to the reaction mixture until the two phases separated. The water phase was extracted with diethyl ether (3 X 50 ml). The combined organic phases were dried with magnesium sulphate and evaporated to give the title product as an oil, 1.43 g (74%). ¹³C NMR (45 MHz) CDCl₃: δ 21.9-43.9 (16 x CH₂), 79.1 (O-CH₂-O), 173.0 (2 X C=O), 202.6 (2 X CHO).

### EXAMPLE 38

### Methylene bis(sulphosuccinimidylazelate) sodium salt

Methylene bis(hydrogen azelate) (0.38 g, 1 mmol), N-hydroxysulphosuccinimide sodium salt (0.48 g, 2.2 mmol) and dicyclohexylcarbodiimide (0.45 g, 2.2 mmol) was dissolved in dimethylformamide (10 ml). The suspension was stirred overnight at room temperature under a nitrogen atmoshphere. The reaction mixture was filtered and purified by reversed phase chromatography (RP-8) with water/acetonitrile (1:1) as eluant to give the title compound.

## Claims

1. A crosslinking agent containing a group of formula (I) 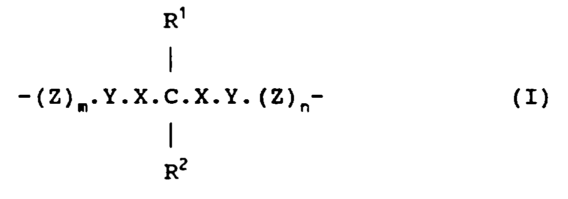 [in which each X, which may be the same or different, is selected from -O-, -S- and -NR-, where R represents a hydrogen atom or an organic group; each Y, which may be the same or different, represents carbonyl, thiocarbonyl, sulphonyl or phosphoryl (i.e. a group of formula 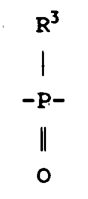 where R³ is a hydrogen atom or an organic group) or a similar acid-forming group; each Z, which may be the same or different, is selected from -O-, -S- and -NR-, where R represents a hydrogen atom or an organic group; m and n, which may be the same or different, are each zero or 1; and R¹ and R, which may be the same or different, are each selected from hydrogen atoms, monovalent organic groups and groups of formula -X.Y.(Z)ₘ- as hereinbefore defined, or R¹ and R together form a divalent organic group] or containing a group adapted to generate a group of formula (I) upon reaction with a reagent or substrate containing a species H.X.Y.(Z)ₘ- or a reactive derivative thereof with the proviso that when the grouping of formula (I) is attached to two optionally substituted lower alk-l-enyl groups, both of X represent -O- or -NR- and both of Y represents -CO-, then at least one of m and n is 1.

2. A crosslinking agent as claimed in claim 1 of the formula (II) 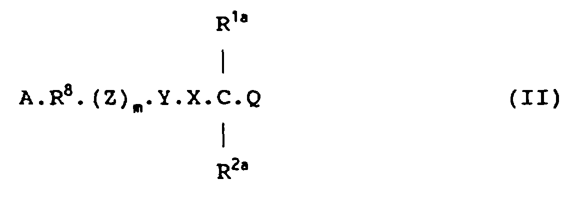 (wherein Q is a leaving group L or a group of formula -X.Y.(Z)ₙ.R⁹.B; X, Y, Z, m and n are as defined in claim 1; R^{1a} and R^{2a} are as defined for R¹ and R in claim 1 except that they may represent groups -X.Y.(Z)ₘ.R⁸.A or -X.Y.(Z)ₙ.R⁹.B rather than groups -X.Y.(Z)ₘ-; R⁸ and R⁹, which may be the same or different, represent divalent organic groups optionally interrupted by one or more heteroatoms and/or carrying one or more substituents containing heteroatoms; and A and B, which may be the same or different, optionally in conjunction with the groups R⁸ and R⁹ to which they are attached, represent functional groupings reactive with the species to be crosslinked; with the proviso that when both A.R⁸- and -R⁹.B represent optionally substituted lower alk-l-enyl groups, both of X represent -O- or -NR- and both of Y represent -CO-, then at least one of m and n is 1).

3. A crosslinking agent as claimed in claim 1 or claim 2 in which each X is -O- and each Y is -CO-.

4. A crosslinking agent as claimed in any of claims 1 to 3 in which R, R¹, R and R³ are each selected from hydrogen atoms and aliphatic, cycloalkyl, araliphatic, aryl and heterocyclic groups.

5. A crosslinking agent as claimed in any of claims 1 to 3 in which R, R¹, R and R³ are selected from hydrogen atoms and C₁₋₄ alkyl groups.

6. A crosslinking agent as claimed in any of claims 2 to 5 in which R⁸ and R⁹ (where present) are selected from alkylene groups, alkenylene groups, cycloalkylene groups, arylene groups, aralkylene groups and heterocyclic groups, any of which may be substituted and/or be interrupted by heteroatoms.

7. A crosslinking agent as claimed in any of claims 2 to 6 in which R⁸ and R⁹ (where present) are each selected from C₁₋₃₀ alkylene optionally interrupted by one or more oxy, carbonyloxy or oxycarbonyl groups; phenylene; phenyleneoxy and phenyleneethynyl groups.

8. A crosslinking agent as claimed in any of claims 2 to 7 in which A or B (where present) are selected from halogen atoms, aryl halides, sulphonyloxy groups, α-halomethyl carbonyl groups, carboxyl groups, activated carboxyl groups, hydroxyl groups, activated hydroxyl groups, mercapto groups, alkene groups, activated alkene groups, conjugated diyne systems, conjugated enyne systems, epoxy groups, acetal-forming aldehyde and ketone groups and derivatives thereof, amino groups, diazo groups, imidoester groups, alkyl and aralkyl halide groups, nitroaryl halide groups, nitrene precursor groups, carbene precursor groups, aldehyde groups, ketone groups, isocyanate groups, isothiocyanate groups, semicarbazide groups, thiosemicarbazide groups, phenol ester groups, acyl azide groups, hydrazine groups, haloformate groups, optionally sulphonated maleimide groups, nitrosourea groups, s-triazine groups, aziridine groups and pyridyl disulphide groups.

9. A crosslinking agent as claimed in claim 8 in which A and B (where present) are O-linked sulphonated N-hydroxysuccinimidyl residues.

10. A crosslinking agent as claimed in claim 2 in which L is a leaving group and -R⁸A terminates in a grouping 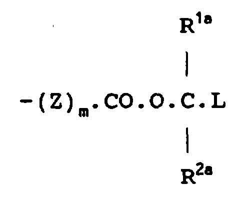 (where m, Z, R^{1a}, R^{2a} and L have the meanings given in claim 2).

11. A crosslinking agent as claimed in any of claims 2 to 10 in which L is a halogen atom.

12. A process for the preparation of a crosslinking agent as defined in claim 2 in which
(A) either one or two equivalents of a compound of formula (V)
A.R⁸.(Z)ₘ.Y.X.H (V)
(where X, Y, Z, m, R⁸ and A are as defined in claim 2, subject if necessary and/or desired to any reactive groups being protected) or a functional derivative thereof are caused to react with one equivalent of a compound of formula (VI) 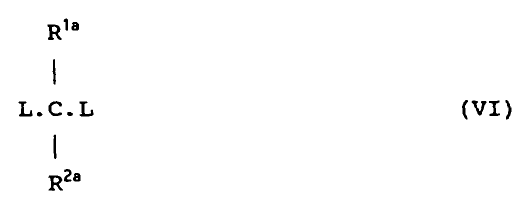 (where R^{1a}, R^{2a} and L are as defined in claim 2 and the substituents L may be the same or different);
(B) one equivalent of a compound of formula (V) as defined in (A) above or a protected and/or functional derivative thereof is caused to react with one equivalent of a compound of formula (VII) 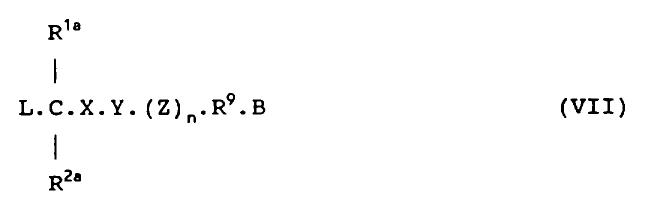 (where X, Y, Z, n, R^{1a}, R^{2a}, B and L are as defined in (A) above, subject if necessary and/or desired to any reactive groups being protected) ;
(C) for the production of symmetrical compounds of formula (II) in which R^{2a} is hydrogen, m and n are zero, each Y represents a carbonyl group and each X represents -O-, a compound of formula (VIII)
A.R⁸.CO.OH (VIII)
(where A and R⁸ are as defined in (A) above, subject if necessary and/or desired to A and any other reactive groups being protected) is caused to react with an aldehyde of formula (IX)
R^{1a}.CHO (IX)
(where R^{1a} is as defined in (A) above) in the presence of an acid catalyst;
(D) for the production of compounds of formula (II) in which L is a halogen atom, a compound of formula (V) as defined in (A) above or a protected and/or functional derivative thereof is caused to react with an aryl thioether of formula (X) 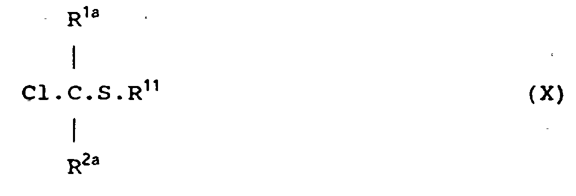 (where R^{1a} and R^{2a} are as defined in (A) above and R¹¹ represents an aryl group) to form a compound of formula (XI) 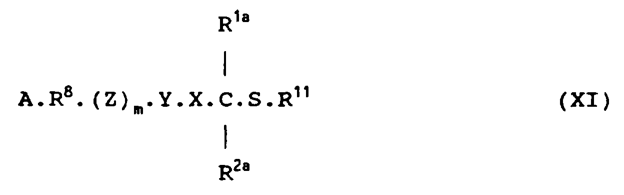 (where X, Y, Z, m, R^{1a}, R^{2a}, R⁸ and R¹¹ are as hereinbefore defined) and the latter compound (XI) is caused to react with a halogenating agent;
(E) for the production of compounds of formula (II) in which Q is a leaving group L, a chlorosulphate of formula (XII) 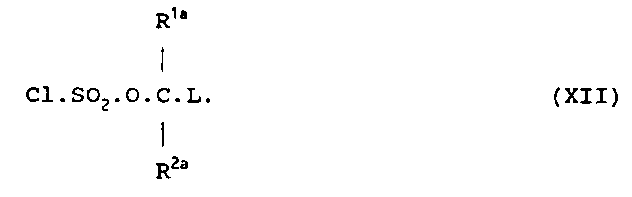 (where R^{1a}, R^{2a} and L are as defined in (A) above) is caused to react which a compound of formula (V) as defined in (A) above or a protected and/or functional derivative thereof;
(F) for the production of compounds of formula (III) in which L is a halogen atom, a compound of formula (XIII) 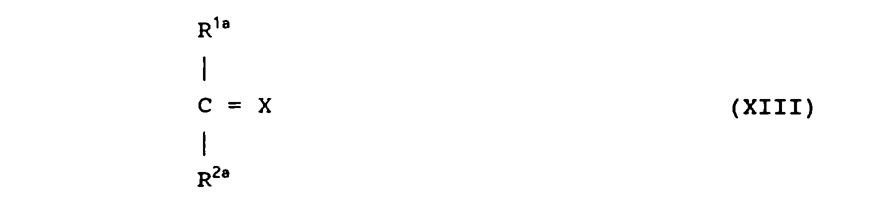 (where R^{1a}, R^{2a} and X have the meanings given in (A) above) is caused to react with a compound of formula (XIV)
Hal.Y.(Z)ₙ.R⁹.B (XIV)
(where Hal is a halogen atom and Y, Z, n, R⁹ and B have the meanings given in (A) above);
followed where necessary and/or desired by removal of protecting groups and/or interconversion of reactive groupings A and/or B.

13. Use of a crosslinking agent as claimed in any of claims 1 to 11, including crosslinking agents not subject to the proviso in claim 1, to prepare substrates containing biodegradable crosslinkages.

14. Use as claimed in claim 13 wherein the substrate is an ultrasound contrast agent.

## Patentansprüche

1. Vernetzungsmittel, enthaltend eine Gruppe der Formel (I) 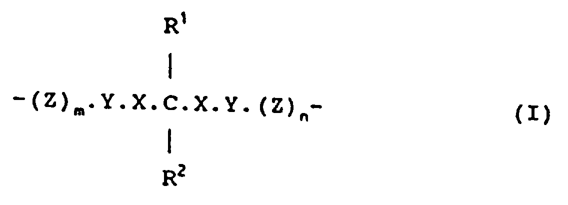 (worin jeder der Reste X, die gleich oder verschieden sein können, ausgewählt ist unter -O-, -S- und -NR-, wobei R ein Wasserstoffatom oder eine organische Gruppe bedeutet; jeder der Reste Y, die gleich oder verschieden sein können, eine Carbonyl-, Thiocarbonyl-, Sulfonyl- oder Phosphoryl- (d.h. eine Gruppe der Formel 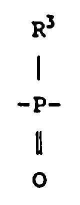 worin R³ für ein Wasserstoffatom oder eine organische Gruppe steht) oder eine ähnliche, säurebildende Gruppe bedeutet; jeder der Reste Z, die gleich oder verschieden sein können, ausgewählt ist unter -O-, -S- und -NR-, wobei R ein Wasserstoffatom oder eine organische Gruppe bedeutet; m und n, die gleich oder verschieden sein können, jeweils 0 oder 1 sind; und R¹ und R, welche gleich oder verschieden sein können, jeweils ausgewählt sind unter Wasserstoffatomen, monovalenten organischen Gruppen und Gruppen der Formel -X.Y.(Z)ₘ-, wie oben definiert, oder R¹ und R zusammen eine divalente organische Gruppe bilden] oder enthaltend eine Gruppe, die zur Bildung einer Gruppe der Formel (I) nach Reaktion mit einem Reagens oder einem Substrat, enthaltend eine Spezies H.X.Y.(Z)ₘ- oder ein reaktives Derivat davon, befähigt ist, mit der Maßgabe, daß wenigstens einer der Parameter m und n gleich 1 ist, wenn die Gruppierung der Formel (I) an zwei gegebenenfalls substituierte Niedrig-alk-1-enylgruppen gebunden ist, beide Reste X für -O- oder -NR- stehen und beide Reste Y für -CO- stehen.

2. Vernetzungsmittel nach Anspruch 1 der Formel (II) 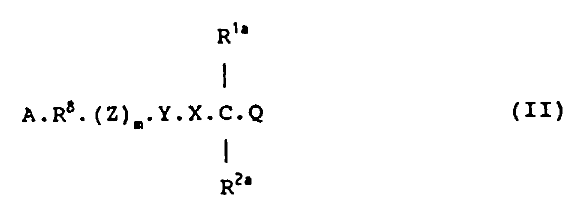 (worin Q für eine Abgangsgruppe L oder eine Gruppe der Formel -X.Y.(Z)ₙ.R⁹,B steht; X, Y, Z, m und n wie in Anspruch 1 definiert sind; R^{1a} und R^{2a} wie R¹ und R in Anspruch 1 definiert sind, mit Ausnahme davon, daß sie für die Gruppen -X.Y.(Z)ₘ.R⁸.A oder -X.Y.(Z)ₙ.R⁹.B an Stelle von Gruppen -X.Y.(Z)ₘ- stehen können; R⁸ und R⁹, welche gleich oder verschieden sein können, für divalente organische Gruppen stehen, die gegebenenfalls durch ein oder mehrere Heteroatome unterbrochen sind und/oder ein oder mehrere Heteroatome enthaltende Substituenten tragen; und A und B, welche gleich oder verschieden sein können, gegebenenfalls in Verbindung mit den Gruppen R⁸ und R⁹, an die sie gebunden sind, für funktionelle Gruppen stehen, die gegenüber den zu vernetzenden Spezies reaktiv sind; mit der Maßgabe, daß wenigstens einer der Parameter m und n gleich 1 ist, wenn beide Reste A.R⁸- und -R⁹.B für gegebenenfalls substituierte Niedrig-alk-l-enylgruppen stehen, beide Reste X für -O- oder -NR- stehen und beide Reste Y für -CO- stehen).

3. Vernetzungsmittel nach Anspruch 1 oder Anspruch 2, worin jedes X für -O- und jedes Y für -CO- steht.

4. Vernetzungsmittel nach einem der Ansprüche 1 bis 3, worin R, R¹, R und R³ jeweils ausgewählt sind unter Wasserstoffatomen und aliphatischen, Cycloalkyl-, araliphatischen, Aryl- und heterocyclischen Gruppen.

5. Vernetzungsmittel nach einem der Ansprüche 1 bis 3, worin R, R¹, R und R³ ausgewählt sind unter Wasserstoffatomen und C₁₋₄-Alkylgruppen.

6. Vernetzungsmittel nach einem der Ansprüche 2 bis 5, worin R⁸ und R⁹ (falls vorhanden) ausgewählt sind unter Alkylengruppen, Alkenylengruppen, Cycloalkylengruppen, Arylengruppen, Aralkylengruppen und heterocyclischen Gruppen, von denen jede durch Heteroatome substituiert und/oder unterbrochen sein kann.

7. Vernetzungsmittel nach einem der Ansprüche 2 bis 6, worin R⁸ und R⁹ (falls vorhanden) jeweils ausgewählt sind unter gegebenenfalls durch ein oder mehrere Oxy-, Carbonyloxy-, oder Oxycarbonylgruppen unterbrochene C₁₋₃₀-Alkylengruppen; Phenylengruppen; Phenylenoxy- und Phenylenethinylgruppen.

8. Vernetzungsmittel nach einem der Ansprüche 2 bis 7, worin A oder B (falls vorhanden) ausgewählt sind unter Halogenatomen, Arylhalogeniden, Sulfonyloxygruppen, α-Halomethylcarbonylgruppen, Carboxylgruppen, aktivierten Carboxylgruppen, Hydroxylgruppen, aktivierten Hydroxylgruppen, Mercaptogruppen, Alkengruppen, aktivierten Alkengruppen, konjugierten Diinsystemen, konjugierten Eninsystemen, Epoxygruppen, acetalbildenden Aldehyd- und Ketongruppen und Derivaten davon, Aminogruppen, Diazogruppen, Imidoestergruppen, Alkyl- und Aralkylhalogenidgruppen, Nitroarylhalogenidgruppen, Gruppen einer Nitrenvorstufe, Gruppen einer Carbenvorstufe, Aldehydgruppen, Ketongruppen, Isocyanatgruppen, Isothiocyanatgruppen, Semicarbazidgruppen, Thiosemicarbazidgruppen, Phenolestergruppen, Acylazidgruppen, Hydrazingruppen, Haloformiatgruppen, gegebenenfalls sulfonierten Maleimidgruppen, Nitrosoharnstoffgruppen, S-Triazingruppen, Aziridingruppen und Pyridyldisulfidgruppen.

9. Vernetzungsmittel nach Anspruch 8, worin A und B (falls vorhanden) für O-verknüpfte sulfonierte N-Hydroxysuccinimidylreste stehen.

10. Vernetzungsmittel nach Anspruch 2, worin L für eine Abgangsgruppe steht und -R⁸A in der Gruppierung 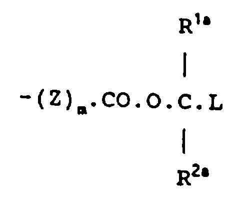 endet (worin m, Z, R^{1a}, R^{2a} und L die in Anspruch 2 angegebenen Bedeutungen besitzen).

11. Vernetzungsmittel nach einem der Ansprüche 2 bis 10, worin L für ein Halogenatom steht.

12. Verfahren zur Herstellung eines Vernetzungsmittels nach Anspruch 2, wobei
(A) entweder 1 oder 2 Äquivalente einer Verbindung der Formel (V)
A.R⁸.(Z)ₘ.Y.X.H (V)
(worin X, Y, Z, m, R⁸ und A, erforderlichenfalls und/oder gewünschtenfalls in Abhängigkeit von reaktiven, zu schützenden Gruppen, wie in Anspruch 2 definiert sind) oder ein funktionelles Derivat davon, mit einem Äquivalent der Verbindung der Formel (VI) 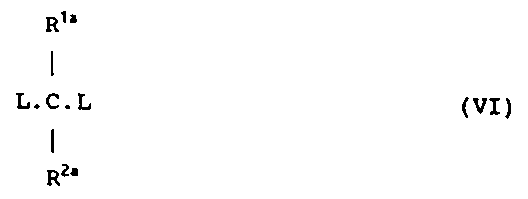 (worin R^{1a}, R^{2a} und L wie in Anspruch 2 definiert sind und die Substituenten L gleich oder verschieden sein können);
zur Reaktion gebracht werden;
(B) ein Äquivalent einer Verbindung der Formel (V), die oben unter (A) definiert ist, oder ein geschütztes und/oder funktionelles Derivat davon mit einem Äquivalent einer Verbindung der Formel (VII) 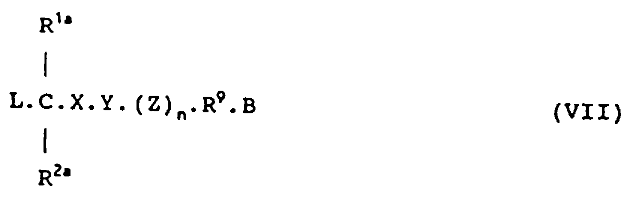 (worin X, Y, Z, n, R^{1a}, R^{2a}, B und L wie oben unter (A), erforderlichenfalls oder gewünschtenfalls in Abhängigkeit von reaktiven, zu schützenden Gruppen, definiert sind)
zur Reaktion gebracht wird;
(C) zur Herstellung symmetrischer Verbindungen der Formel (II), worin R^{2a} für Wasserstoff steht, m und n gleich 0 sind, jedes Y für eine Carbonylgruppe steht und jedes X für -O- steht, eine Verbindung der Formel (VIII)
A.R⁸.CO.OH (VIII)
(worin A und R⁸ wie oben unter (A), erforderlichenfalls und/oder gewünschtenfalls in Abhängigkeit von A und anderen reaktiven, zu schützenden Gruppen, definiert sind) mit einem Aldehyd der Formel (IX)
R^{1a}.CHO (IX)
(worin R^{1a} wie oben unter (A) definiert ist) in Anwesenheit eines sauren Katalysators zur Reaktion gebracht wird;
(D) zur Herstellung von Verbindungen der Formel (II), worin L für ein Halogenatom steht, eine Verbindung der Formel (V), die wie oben unter (A) definiert ist, oder ein geschütztes und/oder funktionelles Derivat davon mit einem Arylthioether der Formel (X) 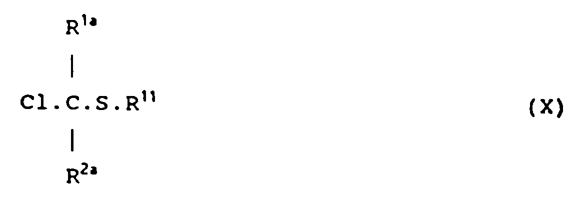 (worin R^{1a} und R^{2a} wie oben unter (A) definiert sind und R¹¹ für eine Arylgruppe steht)
zur Reaktion gebracht wird, um eine Verbindung der Formel (XI) 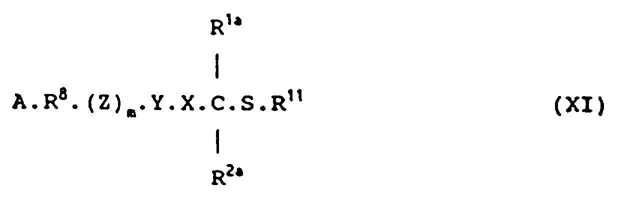 (worin X, Y, Z, m, R^{1a}, R^{2a}, R⁸ und R¹¹ wie vorher definiert sind) zu bilden, und letztere Verbindung (XI) mit einem halogenierenden Agens zur Reaktion gebracht wird;
(E) zur Herstellung von Verbindungen der Formel (II), worin Q für eine Abgangsgruppe L steht, ein Chlorsulfat der Formel (XII) 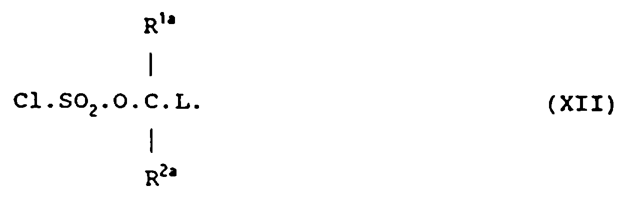 (worin R^{1a}, R^{2a} und L wie oben unter (A) definiert sind) mit einer Verbindung der Formel (V), die wie oben unter (A) definiert ist, oder einem geschützten und/oder funktionellen Derivat davon zur Reaktion gebracht wird;
(F) zur Herstellung von Verbindungen der Formel (II), worin L für ein Halogenatom steht, eine Verbindung der Formel (XIII) 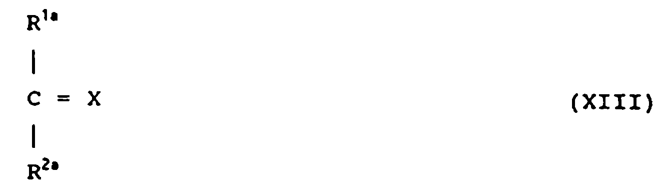 (worin R^{1a}, R^{2a} und X die unter (A) angegebenen Bedeutungen besitzen) mit einer Verbindung der Formel (XIV)
Hal.Y.(Z)ₙ.R⁹.B (XIV)
(worin Hal für ein Halogenatom steht und Y, Z, n, R⁹ und B die oben in (A) genannten Bedeutungen besitzen) zur Reaktion gebracht wird;
und anschließend, falls erforderlich und/oder erwünscht, die Schutzgruppen entfernt werden und/oder die reaktiven Gruppierungen A und/oder B umgewandelt werden.

13. Verwendung eines Vernetzungsmittels nach einem der Ansprüche 1 bis 11, einschließlich der Vernetzungsmittel, die durch den Vorbehalt von Anspruch 1 ausgenommen sind, zur Herstellung von Substraten, die biologisch abbaubare Vernetzungen enthalten.

14. Verwendung nach Anspruch 13, worin das Substrat ein Ultraschallkontrastmittel ist.

## Revendications

1. Agent de réticulation contenant un radical de la formule (I) 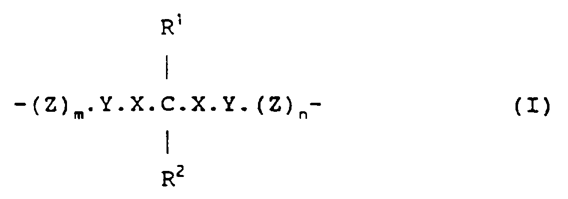 [dans laquelle chaque symbole X, qui peut avoir des significations identiques ou différentes, est choisi parmi -O-, -S- et -NR- où R représente un atome d'hydrogène ou un groupe organique, chaque symbole Y, qui peut avoir des significations identiques ou différentes, représente un radical carbonyle, thiocarbonyle, sulfonyle ou phosphoryle (c'est-à-dire un groupe de la formule 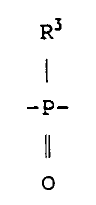 où R³ représente un atome d'hydrogène ou un groupe organique) ou un groupe acidogène similaire, chaque symbole Z, qui peut avoir des significations identiques ou différentes, est choisi parmi -O-, -S- et -NR- où R représente un atome d'hydrogène ou un groupe organique, m et n, qui peuvent être identiques ou différents, représentent chacun un nombre égal à 0 ou à 1 et les symboles R¹ et R, qui peuvent être identiques ou différents, représentent chacun un atome d'hydrogène, un radical organique monovalent ou un groupe de la formule -X.Y.(Z)ₘ-, tel que précédemment défini, ou bien R¹ et R forment en commun un groupe organique divalent], ou contenant un groupe susceptible d'engendrer un groupe de la formule (I) par réaction avec un réactif ou un substrat contenant une espèce H.X.Y.(Z)ₘ- ou un de ses dérivés réactifs, avec la condition que lorsque le groupement de la formule (I) est attaché à deux radicaux alc-1-ényle éventuellement substitués, que les deux symboles X représentent -O- ou -NR- et que les deux symboles Y représentent -CO-, alors au moins l'un des indices m et n soit égal à 1.

2. Agent de réticulation suivant la revendication 1, répondant à la formule (II) 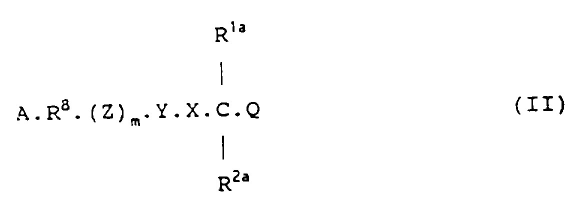 [dans laquelle Q représente un groupe labile ou sortant L, ou un groupe de la formule -X.Y.(Z)ₙ.R⁹.B; X, Y, Z, m et n possèdent les significations qui leur ont été attribuées dans la revendication 1: R^{1a} et R^{2a} sont tels que définis à propos de R¹ et de R dans la revendication 1, à l'exception qu'ils peuvent représenter des groupes -X.Y.(Z)ₘ.R⁸.A ou -X.Y.(Z)ₙ.R⁹.B plutôt que des groupes -X.Y.(Z)ₘ-; les symboles R⁸ et R⁹, qui peuvent être identiques ou différents, représentent chacun un groupe organique divalent, éventuellement interrompu par un ou plusieurs hétéroatomes et/ou portant un ou plusieurs substituants contenant des hétéroatomes; les symboles A et B, qui peuvent être identiques ou différents, représentent, éventuellement conjointement avec les groupes R⁸ et R⁹ auxquels ils sont attachés, des groupements fonctionnels susceptibles de réagir avec l'espèce à réticuler; avec la condition que lorsque les deux ensembles A.R⁸- et -R⁹.B représentent des radicaux alc-1-ényle inférieurs éventuellement substitués, que les deux symboles X représentent -O- ou -NR- et que les deux symboles Y représentent -CO-, alors au moins l'un des indices m et n soit égal à 1].

3. Agent de réticulation suivant la revendication 1 ou la revendication 2, caractérisé en ce que chaque symbole X représente -O- et chaque symbole Y représente -CO-.

4. Agent de réticulation suivant l'une quelconque des revendications 1 à 3, caractérisé en ce que R, R¹, R et R³ sont chacun choisis parmi les atomes d'hydrogène et des radicaux aliphatiques, cycloalkyle, araliphatiques, aryle et hétérocycliques.

5. Agent de réticulation suivant l'une quelconque des revendications 1 à 3, caractérisé en ce que R, R¹, R et R³ sont choisis parmi les atomes d'hydrogène et des radicaux alkyle en C₁ à C₄.

6. Agent de réticulation suivant l'une quelconque des revendications 2 à 5, caractérisé en ce que R⁸ et R⁹ (lorsqu'ils sont présents) sont choisis parmi les radicaux alkylène, les radicaux alcénylène, les radicaux cycloalkylène, les radicaux arylène, les radicaux aralkylène et les radicaux hétérocycliques, n'importe lesquels d'entre eux pouvant être substitués et/ou interrompus par des hétéroatomes.

7. Agent de réticulation suivant l'une quelconque des revendications 2 à 6, caractérisé en ce que R⁸ et R⁹ (lorsqu'ils sont présents) sont chacun choisis parmi les radicaux alkylène en C₁ à C₃₀, éventuellement interrompus par un ou plusieurs groupes oxy, carbonyloxy ou oxycarbonyle, les radicaux phénylène, phénylèneoxy et phényléneéthynyle.

8. Agent de réticulation suivant l'une quelconque des revendications 2 à 7, caractérisé en ce que A et B (lorsqu'ils sont présents) sont choisis parmi les atomes d'halogènes, les halogénures d'aryle, les groupes sulfonyloxy, les groupes α-halométhylcarbonyle, les groupes carboxyle, les groupes carboxyle activés, les groupes hydroxyle, les groupes hydroxyle activés, les groupes mercapto, les groupes alcène, les groupes alcène activés, des systèmes du type diyne conjugué, les systèmes ényme conjugué, les groupes époxy, les groupes cétone et aldéhyde engendrant des fonctions acétal et leurs dérivés, les groupes amino, les groupes diazo, les groupes imidoester, les groupes halogénure d'alkyle et d'aralkyle, les groupes halogénure de nitroaryle, les radicaux précurseurs de nitrènes, les groupes précurseurs de carbènes, les groupes aldéhyde, les groupes cétone, les groupes isocyanate, les groupes isothiocyanate, les groupes semicarbazide, les groupes thiosemicarbazide, les groupes ester phénolique, les groupes acylazide, les groupes hydrazine, les groupes haloformiate, les groupes maléimide éventuellement sulfonés, les groupes nitrozo-urée, les groupes s-triazine, les groupes aziridine et les groupes pyridyldisulfure.

9. Agent de réticulation suivant la revendication 8, caractérisé en ce que A et B (lorsqu'ils sont présents) sont des restes N-hydroxysuccinimidyle sulfonés O-liés.

10. Agent de réticulation suivant la revendication 2, caractérisé en ce que L est un groupe labile ou sortant et -R⁸A se termine par un groupement 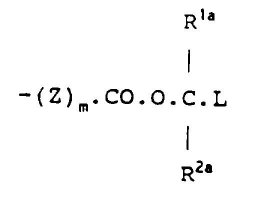 (dans laquelle m, Z, R^{1a}, R^{2a} et L possèdent les significations qui leur ont été attribuées dans la revendication 2).

11. Agent de réticulation suivant l'une quelconque des revendications 2 à 10, caractérisé en ce que L représente un atome d'halogène.

12. Procédé de préparation d'un agent de réticulation tel que défini dans la revendication 2, caractérisé en ce que
(A) on amène à réagir un ou deux équivalents d'un composé de la formule (V)
A.R⁸.(Z)ₘ.Y.X.H (V)
(dans laquelle X, Y, Z, m, R⁸ et A possèdent les significations qui leur ont été attribuées dans la revendication 2, soumis, si cela se révèle nécessaire et/ou souhaitable, à protection de n'importe quels groupes réactifs), ou un dérivé fonctionnel d'un tel composé, avec un équivalent d'un composé de la formule (VI) 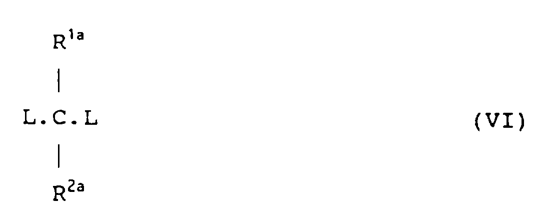 (dans laquelle R^{1a}, R^{2a} et L possèdent les significations qui leur ont été attribuées dans la revendication 2 et les substituants L peuvent être identiques ou différents);
(B) on amène à réagir un équivalent d'un composé de la formule (V) telle que définie en (A) ci-dessus, ou un dérivé protégé et/ou fonctionnel d'un tel composé, avec un équivalent d'un composé de la formule (VII) 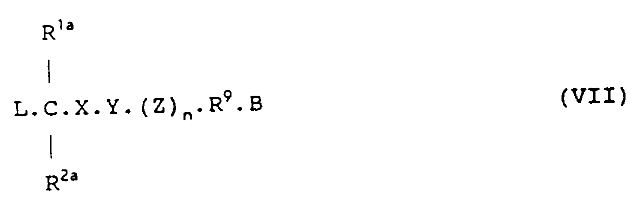 (dans laquelle X, Y, Z, n, R^{1a}, R^{2a}, B et L possèdent les significations qui leur ont été attribuées en (A) ci-dessus, soumis, si cela se révèle nécessaire et/ou souhaitable, à une protection de n'importe quels groupes réactifs);
(C) en vue de la production de composés symétriques de la formule (II) dans laquelle R^{2a} représente un atome d'hydrogène, m et n sont égaux à 0, chaque symbole Y représente un groupe carbonyle et chaque symbole X représente -O-, on amène à réagir un composé de la formule (VIII)
A.R⁸.CO.OH (VIII)
(dans laquelle A et R⁸ possèdent les significations qui leur ont été attribuées en (A) ci-dessus, soumis, si cela se révèle nécessaire et/ou souhaitable, à une protection de A et de n'importe quels autres groupes réactifs) avec un aldéhyde de la formule (IX)
R^{1a}.CHO (IX)
(dans laquelle R^{1a} possède les significations qui lui ont été attribuées en (A) ci-dessus) en présence d'un catalyseur acide;
(D) en vue de la production de composés de la formule (II) dans laquelle L représente un atome d'halogène, on amène à réagir un composé de la formule (V) telle que définie en (A) ci-dessus, ou un dérivé protégé et/ou fonctionnel d'un tel composé, avec un arylthioéther de la formule (X) 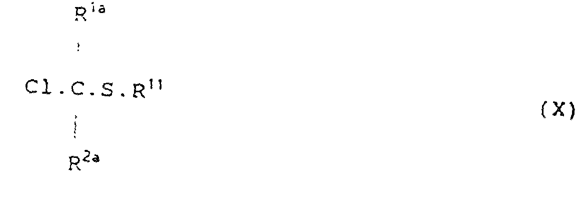 (dans laquelle R^{1a} et R^{2a} possèdent les significations qui leur ont été attribuées en (A) ci-dessus et R¹¹ représente un radical aryle), pour former un composé de la formule (XI) 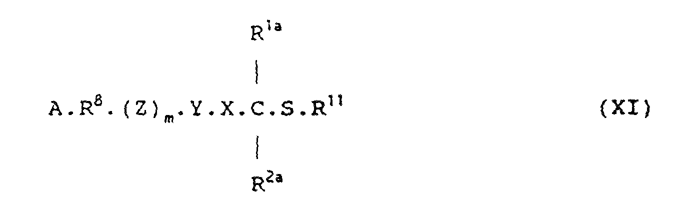 (dans laquelle X, Y, Z, m, R^{1a}, R^{2a}, R⁸ et R¹¹ possèdent les significations qui leur ont été précédemment attribuées) et on amène ce dernier composé (XI) à réagir avec un agent d'halogénation;
(E) en vue de la production de composés de la formule (II) dans laquelle Q représente un groupe sortant ou labile L, on amène à réagir un chlorosulfate de la formule (XII) 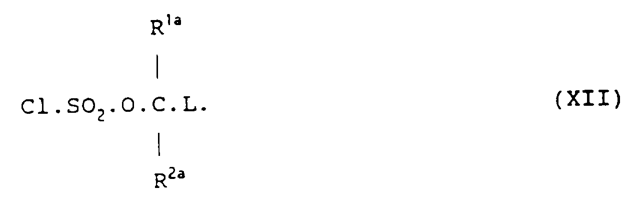 (dans laquelle R^{1a}, R^{2a} et L possèdent les significations qui leur ont été attribuées en (A) ci-dessus) avec un composé de la formule (V) telle que définie en (A) ci-dessus, ou un dérivé protégé et/ou fonctionnel d'un tel composé;
(F) en vue de la production de composés de la formule (II) dans laquelle L représente un atome d'halogène, on amène à régir un composé de la formule (XIII) 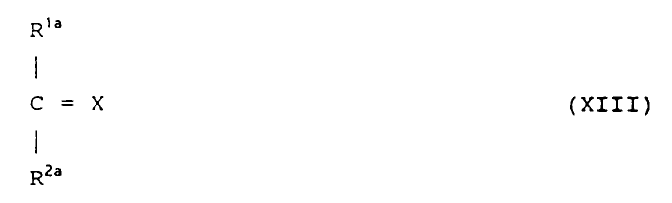 (dans laquelle R^{1a}, R^{2a} et X possèdent les significations qui leur ont été attribuées en (A) ci-dessus) avec un composé de la formule (XIV)
Hal.Y.(Z)ₙ.R⁹.B (XIV)
(dans laquelle Hal représente un atome d'halogène et Y, Z, n, R⁹ et B possèdent les significations qui leur ont été attribuées en (A) ci-dessus);
puis, si cela se révèle nécessaire et/ou souhaitable, on entreprend l'élimination des groupes protecteurs et/ou l'interconversion des groupements réactifs A et/ou B.

13. Utilisation d'un agent de réticulation suivant l'une quelconque des revendications 1 à 11, y compris des agents de réticulation non soumis à la condition exprimée dans la revendication 1, en vue de la préparation de substrats ou supports contenant des liaisons réticulées biodégradables.

14. Utilisation suivant la revendication 13, caractérisée en ce que le substrat ou le support est un agent de contraste pour ultrasons.
